# EUROPEAN PATENT SPECIFICATION

(11) **EP 2 417 261 B1**
(45) Date of publication and mention of the grant of the patent: **05.08.2015**
(21) Application number: 10701594.3
(22) Date of filing: 20.01.2010
(51) Int. Cl.: C12N 15/82, A01H 5/10, C12N 15/11, C12Q 1/68

(54) **MARKERS ASSOCIATED WITH SOYBEAN RUST RESISTANCE AND METHODS OF USE THEREFOR**
MIT SOJABOHNENROSTRESISTENZ ASSOZIIERTE MARKER UND VERFAHREN ZU IHRER VERWENDUNG
MARQUEURS ASSOCIÉS À LA RÉSISTANCE DU SOJA À LA ROUILLE ET LEURS PROCÉDÉS D'UTILISATION

(30) Priority: 18.02.2009 US 153495 P; 17.07.2009 WO PCT/US2009/051003
(43) Date of publication of application: 15.02.2012
(73) Proprietor: Syngenta Participations AG, 4058 Basel (CH)
(72) Inventor: YU, Ju-kyung, Research Triangle Park, NC 27709-2257 (US); BREITINGER, Becky, Research Triangle Park, NC 27709-2257 (US); BOWERS, Glenn, Research Triangle Park, NC 27709-2257 (US); CHAKRABORTY, Nanda, Research Triangle Park, NC 27709-2257 (US)
(74) Representative: Syngenta International AG
(86) International application number: PCT/US2010/021523
(87) International publication number: WO 2010/096227

(56) References cited:
- WO-A-2009/079729
- WO-A2-2010/009404
- US-A1- 2006 288 444
- ALEXANDRE GARCIA ET AL: "Molecular mapping of soybean rust (Phakopsora pachyrhizi) resistance genes: discovery of a novel locus and alleles" THEORETICAL AND APPLIED GENETICS ; INTERNATIONAL JOURNAL OF PLANT BREEDING RESEARCH, SPRINGER, BERLIN, DE, vol. 117, no. 4, 28 May 2008 (2008-05-28), pages 545-553, XP019618398 ISSN: 1432-2242
- DANIELLE C G SILVA ET AL: "Molecular mapping of two loci that confer resistance to Asian rust in soybean" THEORETICAL AND APPLIED GENETICS ; INTERNATIONAL JOURNAL OF PLANT BREEDING RESEARCH, SPRINGER, BERLIN, DE, vol. 117, no. 1, 8 April 2008 (2008-04-08) , pages 57-63, XP019618352 ISSN: 1432-2242
- HYTEN D L ET AL: "Map location of the Rpp1 locus that confers resistance to soybean rust in soybean" THE PLANT GENOME, CROP SCIENCE SOCIETY OF AMERICA, MADISON, WI, US, vol. 47, no. 2, 1 April 2007 (2007-04-01), pages 837-840, XP009102208 ISSN: 0011-183X
- JEFFERY D RAY ET AL: "Genetics and mapping of adult plant rust resistance in soybean PI 587886 and PI 587880A" THEORETICAL AND APPLIED GENETICS ; INTERNATIONAL JOURNAL OF PLANT BREEDING RESEARCH, SPRINGER, BERLIN, DE, vol. 119, no. 2, 26 April 2009 (2009-04-26), pages 271-280, XP019698403 ISSN: 1432-2242

### SEQUENCE LISTING

<110> Syngenta Participations AG Yu, Ju-Kyung Bowers, Glenn Breitinger, Becky Chakraborty, Nanda
<120> MARKERS ASSOCIATED WITH SOYBEAN RUST RESISTANCE AND METHODS OF USE THEREFOR
<130> 1392/64/2 PCT
<150> US 61/153,495
   <151> 2009-02-18
<160> 94
<170> PatentIn version 3.5
<210> 1
   <211> 459
   <212> DNA
   <213> Glycine max
<220>
   <221> misc_feature
   <222> (428)..(428)
   <223> r is a or g
<400> 1
   ttttgatcca aaacaaagct gaaaagaaag gggacaggta tgaagcaatc ttcagcttct      60
   actttggaga ctatggtcac atagcagtgc agggacctta cctgacctat gaggacacat     120
   atttggctgt gactggtggg tctggcatat ttgagggtgt taaaggtcaa gtgaagctgc     180
   gtcagattgt gtatcctttc aagattttgt acacatttta tctaaagggt atcaaggatt     240
   tgcctcagga gcttcttgtc aagactgttg agccaattcc atctgttgaa ccttcccctg     300
   ctgctaaggc ccttgagccc aatgctacca ttgctggctt caccgactaa ttcatcaact     360
   ttttttgtat ttgctttggc ctttgtagta gtatgattta agttactgaa taataataac     420
   aagtgggrac tatgatgggt tttgtagtgg tggagtttc                            459
<210> 2
   <211> 1101
   <212> DNA
   <213> Glycine max
<220>
   <221> misc_feature
   <222> (255)..(932)
   <223> r is a or g
<220>
   <221> misc_feature
   <222> (267)..(267)
   <223> s is c or g
<400> 2
   ttacaccaga atcatggcca ccaatcaaat acccctatca tcttattaca gaaaagggga 60
   taagagaagg ggaaaaaaaa tctctagaaa ctgaagaatc aaggtttggt tcaaaaactg 120
   tgcagttgcg ttagcatgtg aacaagctca tcactagaag gcctatctgc aggcatatca 180
   gagaggcatg cagcagcaat cctaaccgcc attagcatct catcttcctc accttcttcc 240
   cctaacatgc tcttrtctag agcttcstgc gcctcgcccg cttgctgcaa gtgtctcagc 300
   caacatccca aacttccccc actggctgct tctccaaaga atggatctgt aggatcctta 360
   ccagttaaca aaacacctag tatcatgcca aaactaaaga tgtcactttt gtcagtgtac 420
   ctgctgaaat tgccaaaaaa caccattcaa tctgaatacc atgaagctcc acatatacaa 480
   gaatggaatg aagttaaaat aaacatctta ttggcaatac catacccata tatgcaatgc 540
   catcaatcaa gcaatctttt attgttatta cttattgcta ttgatattga tgcatccaga 600
   tatacaccac tgaaaaacta gaagtattcc aagttaaatt aggaaaaaaa acctttgtca 660
   attattatta taatttagtt gtggtctcac tcaccaatct aggtttagta gtttgcagca 720
   tgtgaactat aaactatatt attcatttgg accagactta gtgccaactg cctaaggtct 780
   aaaacttgac atcaggaagt aatgggtagt tacaaaaaca aatagcaaag tctttattaa 840
   ataataccaa attcaatcag caaatgatac aatgacaata cagattacag aatartaatt 900
   tcgtttcaaa acaagagtct ttcattaggg trctacaaag ggggcaaaaa accaatgagg 960
   tgtggagctt ttgaaaaggt aacgaatcaa acaaattccc aatatcaacc acctaattag 1020
   cctattggtg taaaaaagtg aaagaacaga aacagactaa gggaaaacac tagtgaatga 1080
   gtatgattga aatttgtctt a 1101
<210> 3
   <211> 433
   <212> DNA
   <213> Glycine max
<220>
   <221> misc_feature
   <222> (1)..(433)
   <223> r is a or g
<220>
   <221> misc_feature
   <222> (1)..(433)
   <223> y is c or t
<400> 3
   ttgggagcca acctgagggg actcagttac gcaagttttg aagctgttgg ttatgayttt 60
   aatgacatgg ctctaggtga acttgtggaa aggrttttrg cttgcttttg tccagcagag 120
   ttttctgttg ctttgcacat tgacatgcat ggtgagaaac ttaataaatt tcccttrgac 180
   aycaaaggat actactgtgg tgagaggagc actgaagagc ttggagtagg tggtgcagtt 240
   gtgtaccaaa catttgttca aggctgcgat ggtagtgcat ctcctaggtc tattctgaaa 300
   tgctgctgga gtgaggacga gaacgaagat gaagtgaggg agatataagt acttctggtt 360
   tttatgttct gttgttattt atctttactg tcgagtgtgt tattacctaa ataaataaat 420
   atggngggtc gca 433
<210> 4
   <211> 471
   <212> DNA
   <213> Glycine max
<220>
   <221> misc_feature
   <222> (1)..(471)
   <223> r is a or g
<400> 4
   ccctatcctc aaatacaagt ccctgatctc atcataccta agttgttaaa gaaaatctag 60
   cccattccat ataaatctta ttaatacctt actccaggac ctgaaagaca acctacccaa 120
   ctatcattaa acaatttagt ctataccacc tatgcccaaa tagatataga tatatagaaa 180
   caaaccaggt ggaagaggaa taacagagaa gtrtataaaa taaatatgca acttcagact 240
   tgtatgctca agccgcaagc ttatatttga agatgtctat cccacaattc cagatccacc 300
   taaacaagaa taaaaatcca caaaacatta gaagcgaatt cgctgctgca aattagaata 360
   taaaattctt aaaaataatc cttaccaaaa gattcatcac attaataata gtatccatgc 420
   ccatgtgagc catcaccccc rtaataagat gaatatgaac ctctaccata a 471
<210> 5
   <211> 489
   <212> DNA
   <213> Glycine max
<220>
   <221> misc_feature
   <222> (1)..(489)
   <223> m is a or c
<220>
   <221> misc_feature
   <222> (1)..(489)
   <223> w is a or t
<400> 5
   atctcaatct gctttgacag atagagagaa ggagaatctt gacaacttag ttgacaagga      60
   gtgtgcaacm acagagggta tgatatcgag ccagctcaag ttgaagtctt ctttgtcatc     120
   caaagcgtct tctcatcaaa gcatggacaa acatgcaatc cttcgacgta tcaggcaacg     180
   caggtttaat aacaaagcca aaaccgctct ggaagctctc ataggcacct cagaagccaa     240
   caataccggc actgcccaag aacagaagtg gcttcaactg ggtgactctt tctcatctcc     300
   ttaactggaa acacaatctc cccccagttt gcaacccatc tataaatwcc ctctttagtt     360
   tccttcaccc aacaaagtca tcttttccat acgcttgcat ggctaggaaa ggaaatccaa     420
   cccgatcttt atcggcatgc ttaagaaatc ccactatgta tctattaact gtagtagacc     480
   tggtcggtt                                                             489
<210> 6
   <211> 794
   <212> DNA
   <213> Glycine max
<220>
   <221> misc_feature
   <222> (1)..(794)
   <223> r is a or g
<220>
   <221> misc_feature
   <222> (1)..(794)
   <223> w is a or t
<400> 6
   ccaaccacgc tgattcttta ggccatcagt caacccttta ctctggaaac tgctaaggaa      60
   tataaacctt gaaccagaca tgtcccctga agagtagaaa aatatcatgt ttcagcagca     120
   tggttcaaga aaatttgttc atgtaggcaa aattgatttt gaaagaaatt gattatgcaa     180
   aattaatgtt ggttaagatt aattttaaag taatgtgatt tatgtttgaa tattttttat     240
   cgtagcaact cagtataact tttttaattc aatgcaaaaa tattcaaagt tattttgact     300
   caatcaattc tagattcaat atcaattcct catcattgaa acaaatccca acatgtattt     360
   aaaatcacga tagttgatat ttcaacgtaa tttcagaaaa atcaacataa aactaaccac     420
   gcatcactta atgcgtgcta gggtgagcat taaaattgat ttcaaataaa attaattttc     480
   taaaattggt tttggtaaaa attgattttg aagtgacatg agttatgttt gaacgttttt     540
   tattctaaaa gttaataaca aatttcaawt tttttttatc caaagcaaaa gtcatctaaa     600
   agttatttga actcgaaatc aaatttaaac tcaaaatcaa ttccttaatg tgaaattaaa     660
   catgtgtcca tttacctcaa atcatgttat ccgatatttc aacaagactt taaaraatcc     720
   tacgtgaaac caaacatgca cttactagaa taagcagagt gaatagcaga aatacccaga     780
   ttgtaagctg caga                                                       794
<210> 7
   <211> 568
   <212> DNA
   <213> Glycine max
<220>
   <221> misc_feature
   <222> (1)..(568)
   <223> r is a or g
<220>
   <221> misc_feature
   <222> (1)..(568)
   <223> w is a or t
<400> 7
   gttttacaca gttgcacaaa gtcataaact tcagtgacca ttttttatat tagcctggca 60
   gtaagatggc tatttatttc tgagtgtagt ttgtactgat ttaatggttt tattatatgc 120
   ctagactttt ctttgatatc aatatcttat gtcatcttct cttggttgtg gatggtattt 180
   ttcttctgtg gtgtawtttg ttaaaccaat gatattgtat attaattact tttgtaattt 240
   tgatctagta atctattttc atggctattt taaaatattg tagtgggttg aggggaaaat 300
   atttagggaa attatttagg ttgactgtgc cttgcctttt aggaatgcat aacgactttt 360
   tttcttctaa cagttgratt atatacttag ctttgtaatt aattttgttt arttattcaa 420
   ccgatttttt tcccacttta ttagcattta tcatgatctg gttccttcac tatattttgt 480
   ttctgctagt gccaattttt tacctcaaga ctttactttt gtagcttttg tgctgtttat 540
   ttgtttctgt ggatttgtct cgattcat 568
<210> 8
   <211> 503
   <212> DNA
   <213> Glycine max
<220>
   <221> misc_feature
   <222> (1)..(503)
   <223> r is a or g
<220>
   <221> misc_feature
   <222> (1)..(503)
   <223> w is a or t
<220>
   <221> misc_feature
   <222> (1)..(503)
   <223> y is c or t
<400> 8
   ccgagacgrg tgaggaaatc ctcgaggtgg gtcaaggacc cctcgtgctt gcttgggtcg 60
   aacatggagg caacgagagt acaaaatgtt agggacacar taagctggtt ttygaggaac 120
   tagtacctcc agcaattcaa taaagaagaa gaagaacaaa gagggaaaga gtgaggtaat 180
   tggaaatttc attcatgcct tgcttgttat ttacattagt atttataaac ttcctagtaa 240
   cagaaagatc ctaggaatat ttgcccataa caaaatttgt acacattgtc ccctaggagc 300
   agacaatatc aaccaaggaa attctagaag gtagcctcag ctggtctcac ccwtcttcca 360
   aaaacgtact ctttraggta agaaggtttt gtaattcttc tttcgaactt agcttcttcc 420
   tgtaccccct catttgcttt tgtgacccct gcccttgcct ctgtttgtaa gctcgtatca 480
   acatrtttcc gatacagatg cag 503
<210> 9
   <211> 769
   <212> DNA
   <213> Glycine max
<220>
   <221> misc_feature
   <222> (1)..(769)
   <223> m is a or c
<220>
   <221> misc_feature
   <222> (1)..(769)
   <223> r is a or g
<220>
   <221> misc_feature
   <222> (1)..(769)
   <223> s is c or g
<220>
   <221> misc_feature
   <222> (1)..(769)
   <223> w is a or t
<220>
   <221> misc_feature
   <222> (1)..(769)
   <223> y is c or t
<400> 9
   aacagagttg gctataacaa ggttttggat aatgccaagc ccatgaatga tccagatgga      60
   aggcactttt catcttttac ttacctgagg cttagttcac ttctcatgga acgacaaaac     120
   ttcatagagt ttgaaagatt tgtcaaraga atgcatggta agttgatgaa gaaaatttta     180
   aatgtatcty tagcctctaa ttgatttatg aaaatatcta ttaaggaaag aaaatcatcc     240
   ctttatctgg ccttgattga atgaatgctc acgtgtcatc tgraaccttc ttactccaca     300
   gtcccacact cagtagggta aatgggtaat carcaaaacc ctgaagaccc aaatttcttt     360
   cagaggcatg gttcagcaac tgtactaata tcatattgaa ctttggattc cattattcgm     420
   tcccactgat agtgttcttt gatccacctw gcactagtct ttttatctga ttggaatcat     480
   tttataaata attgaacacg aaasgagtat taatgtcagt tttaagaggt gtacttctct     540
   tggagagaaa aacactgaaa ctttaagtag ctgcaaaagt tgttgctatc ttgtgggaca     600
   ttgattatat tagggttgat gactgtgtca tttctcattg caggggaagc tgttcttgat     660
   cttcaggtat aaccggtggg aaacaaagtc ttgttgaatt tgtatttata agtcccctga     720
   caaaatggtt gggggattct gttatagaat aggagaaata ggattgtta                 769
<210> 10
   <211> 513
   <212> DNA
   <213> Glycine max
<220>
   <221> misc_feature
   <222> (1)..(513)
   <223> m is a or c
<220>
   <221> misc_feature
   <222> (1)..(513)
   <223> r is a or g
<400> 10
   gagaaagaga agtattgcgc atcatgccca aatgtggcca cacttttcat ctttcttgca       60
   ttgatatatg gctgaggaaa caatccacct gtccagtatg ccgtctgccg ttgaaaaact      120
   cttccgaaac gaaacatgtg agacctgtga catttaccat gagccaatcc cttgacgagt      180
   ctcacacatc agacagaaac gatgmtattg agagatatgt tgaacctaca cctactgcag      240
   ccagtaactc tttacaacca acttcaggag aacaagaagc aagrcaatga tcttagagaa      300
   ctaaaggggt tgttctgctc aaaaagagaa gaatgtagaa tttctgcttc tatagaggaa      360
   tgcttctaat tatagattgg attcaaattc tttgtctgta atatggcctt catattcact      420
   tggtggtgta aatatgtttc cttttgtagc atatgcgggc caaggttttg gtggaatttc      480
   ttgcataccg atttgaagtt cttttgtcta tgg                                   513
<210> 11
   <211> 281
   <212> DNA
   <213> Glycine max
<220>
   <221> misc_feature
   <222> (1)..(281)
   <223> y is c or t
<400> 11
   atccaccaac accacttcac caccaaagcc tagtggagct gtaactgttt ccagtggcat       60
   tggccttttg gtggggtctt tctttgtgtc agcaattatg tytggtttca tgggctaggg      120
   aagaggcagt gctgtttttt tttttattct cctttttatt aatatgttat tgttttgctg      180
   agagtttgtg tgaatttcat catttaatcc agatgtatat ctaatcttga tatgtatctc      240
   cttttaatat ttaaaaatga cacatttctt tagctgcttc c                          281
<210> 12
   <211> 948
   <212> DNA
   <213> Glycine max
<220>
   <221> misc_feature
   <222> (1)..(948)
   <223> w is a or t
<400> 12
   tgaatttttg ttatgagagc atctccattt taaagagatt tcgtttctgg gtatcagaaa        60
   acacctccct tccgattaga tttctatatt ttgcctagac gtcaaatgca cgatgaattc       120
   tttgtatgat gtttgaaaat tgaagtcttc tgtaatacwt gattatcaac ctcaaatttt       180
   gatgcgacaa caagttatgc tttgtgaant tgttattgat gattgtatgg tgtgatttgg       240
   tttcaggttt gaaacttgtt actgtggacc gtccttttgc tgagaagcac tatgctgatt       300
   tgtctgccaa gcctttcttt agtggtttgg tggattacat tatctctggc cctgttgttg       360
   ccatgatctg ggagggcaag aatgttgtta caactggccg aaagattatn ggagccacaa       420
   acccagctca atctgagcct ggaactatcc gtggtgactt tgccattgac attggaaggt       480
   atagacttgt tttcatcatc gttcaagcaa gtttacgttt tcaaatcttt tgttttaaca       540
   catgaatatg aatctgtttt cattgttgtt catgtttcta atctagtttg gtggatgtta       600
   ggttttcatt nttgttgttt gattttcaat ttctcccgaa tttttttctt atttacaaca      660
   ttggtttcca acgttctgtg ttttagagaa aagagtgttg tgctcaacca attctgcgaa       720
   tattgcaata tgttcatgac atcatcaatt aagcaagatg ataacactgt tgagtacatc       780
   ataatcaagg gagtggaatc caacaaatcc atgttctgta tcttgtattc atgaaaatca       840
   aaattgttgg ggtagtcaat gtttatcaac attaaaaggt agtgtgnatc tanatactta       900
   ttatttncaa ttctataaag gatttgaagg gaactttgaa tttttttt                    948
<210> 13
   <211> 485
   <212> DNA
   <213> Glycine max
<220>
   <221> misc_feature
   <222> (1)..(485)
   <223> r is a or g
<400> 13
   ttctatgacc aacctaccgc ctgtgggtaa agctaaacta agttctcaca ggtgaggtga        60
   gttcaccaac cacggccgcc agaatttcca gatccccatc cgttccctga tgctgctttc       120
   ttgggggcag atccccagcc agagcttcct tgttcaccat cacttggacc agcaccacca       180
   ctcgcttgtc ccccccagcc accattgtca ctgttactag ctccacctcc tccccctccc       240
   caaccactac tgcctccacc gcttccacca ccaccacccc aaccaccagg aaaagcttcc       300
   cttccaggag aattctgaac cttggcccct ggaaaattgc ttaaaccatc atccgartcc       360
   tttgtattat tagaacccca tctgccccca taaccagagt cctgcctttc attattggag       420
   ttgtctcctc tattgttata agaacctcga ccacgaccac gtacacgccc ttttccaccc       480
   accga 485
<210> 14
   <211> 20
   <212> DNA
   <213> Glycine max
<400> 14
   aggatttgcc tcaggagctt 20
<210> 15
   <211> 22
   <212> DNA
   <213> Glycine max
<400> 15
   gaaactccac cactacaaaa cc 22
<210> 16
   <211> 16
   <212> DNA
   <213> Glycine max
<400> 16
   agtggggact atgatg 16
<210> 17
   <211> 17
   <212> DNA
   <213> Glycine max
<400> 17
   agtgggaact atgatgg 17
<210> 18
   <211> 31
   <212> DNA
   <213> Glycine max
<400> 18
   caatcagcaa atgatacaat gacaatacag a 31
<210> 19
   <211> 22
   <212> DNA
   <213> Glycine max
<400> 19
   agctccacac ctcattggtt tt 22
<210> 20
   <211> 19
   <212> DNA
   <213> Glycine max
<400> 20
   tttcattagg gtgctacaa 19
<210> 21
   <211> 21
   <212> DNA
   <213> Glycine max
<400> 21
   tctttcatta gggtactaca a 21
<210> 22
   <211> 22
   <212> DNA
   <213> Glycine max
<400> 22
   caaaagctcc acacctcatt gg 22
<210> 23
   <211> 25
   <212> DNA
   <213> Glycine max
<400> 23
   ttgacatcag gaagtaatgg gtagt 25
<210> 24
   <211> 22
   <212> DNA
   <213> Glycine max
<400> 24
   ttgaaacgaa attattattc tg 22
<210> 25
   <211> 21
   <212> DNA
   <213> Glycine max
<400> 25
   tgttttgaaa cgaaattact a 21
<210> 26
   <211> 23
   <212> DNA
   <213> Glycine max
<400> 26
   gggactcagt tacgcaagtt ttg 23
<210> 27
   <211> 21
   <212> DNA
   <213> Glycine max
<400> 27
   gtgctcctct caccacagta g 21
<210> 28
   <211> 19
   <212> DNA
   <213> Glycine max
<400> 28
   aagctgttgg ttatgattt 19
<210> 29
   <211> 18
   <212> DNA
   <213> Glycine max
<400> 29
   agctgttggt tatgactt 18
<210> 30
   <211> 20
   <212> DNA
   <213> Glycine max
<400> 30
   atcccacaat tccagatcca 20
<210> 31
   <211> 26
   <212> DNA
   <213> Glycine max
<400> 31
   ttatggtaga ggttcatatt catctt 26
<210> 32
   <211> 16
   <212> DNA
   <213> Glycine max
<400> 32
   agccatcacc cccgta 16
<210> 33
   <211> 16
   <212> DNA
   <213> Glycine max
<400> 33
   agccatcacc cccata 16
<210> 34
   <211> 23
   <212> DNA
   <213> Glycine max
<400> 34
   agtctatacc acctatgccc aaa 23
<210> 35
   <211> 22
   <212> DNA
   <213> Glycine max
<400> 35
   ggcttgagca tacaagtctg aa 22
<210> 36
   <211> 20
   <212> DNA
   <213> Glycine max
<400> 36
   aggaataaca gagaagtgta 20
<210> 37
   <211> 22
   <212> DNA
   <213> Glycine max
<400> 37
   aggaataaca gagaagtata ta 22
<210> 38
   <211> 25
   <212> DNA
   <213> Glycine max
<400> 38
   agagagaagg agaatcttga caact 25
<210> 39
   <211> 22
   <212> DNA
   <213> Glycine max
<400> 39
   acgtcgaagg attgcatgtt tg 22
<210> 40
   <211> 16
   <212> DNA
   <213> Glycine max
<400> 40
   aggagtgtgc aaccac 16
<210> 41
   <211> 17
   <212> DNA
   <213> Glycine max
<400> 41
   aggagtgtgc aacaaca 17
<210> 42
   <211> 20
   <212> DNA
   <213> Glycine max
<400> 42
   ccccagtttg caacccatct 20
<210> 43
   <211> 23
   <212> DNA
   <213> Glycine max
<400> 43
   ccatgcaagc gtatggaaaa gat 23
<210> 44
   <211> 17
   <212> DNA
   <213> Glycine max
<400> 44
   ctaaagaggg aatttat 17
<210> 45
   <211> 20
   <212> DNA
   <213> Glycine max
<400> 45
   aaactaaaga gggtatttat 20
<210> 46
   <211> 29
   <212> DNA
   <213> Glycine max
<400> 46
   gtgtccattt acctcaaatc atgttatcc 29
<210> 47
   <211> 24
   <212> DNA
   <213> Glycine max
<400> 47
   gtaagtgcat gtttggtttc acgt 24
<210> 48
   <211> 18
   <212> DNA
   <213> Glycine max
<400> 48
   aagactttaa agaatcct 18
<210> 49
   <211> 19
   <212> DNA
   <213> Glycine max
<400> 49
   caagacttta aaaaatcct 19
<210> 50
   <211> 22
   <212> DNA
   <213> Glycine max
<400> 50
   taaatgctaa taaagtggga aa 22
<210> 51
   <211> 16
   <212> DNA
   <213> Glycine max
<400> 51
   aggttgactg tgcctt 16
<210> 52
   <211> 21
   <212> DNA
   <213> Glycine max
<400> 52
   agctaagtat ataatccaac t 21
<210> 53
   <211> 22
   <212> DNA
   <213> Glycine max
<400> 53
   agctaagtat ataattcaac tg 22
<210> 54
   <211> 25
   <212> DNA
   <213> Glycine max
<400> 54
   ggaggcaacg agagtacaaa atgtt 25
<210> 55
   <211> 25
   <212> DNA
   <213> Glycine max
<400> 55
   tgaattgctg gaggtactag ttcct 25
<210> 56
   <211> 16
   <212> DNA
   <213> Glycine max
<400> 56
   cagcttactg tgtccc 16
<210> 57
   <211> 17
   <212> DNA
   <213> Glycine max
<400> 57
   ccagcttatt gtgtccc 17
<210> 58
   <211> 22
   <212> DNA
   <213> Glycine max
<400> 58
   gaacatggag gcaacgagag ta 22
<210> 59
   <211> 29
   <212> DNA
   <213> Glycine max
<400> 59
   ttcttcttta ttgaattgct ggaggtact 29
<210> 60
   <211> 16
   <212> DNA
   <213> Glycine max
<400> 60
   ttcctcaaaa accagc 16
<210> 61
   <211> 15
   <212> DNA
   <213> Glycine max
<400> 61
   tcctcgaaaa ccagc 15
<210> 62
   <211> 23
   <212> DNA
   <213> Glycine max
<400> 62
   tttcataaat caattagagg cta 23
<210> 63
   <211> 19
   <212> DNA
   <213> Glycine max
<400> 63
   gaggcttagt tcacttctc 19
<210> 64
   <211> 16
   <212> DNA
   <213> Glycine max
<400> 64
   accatgcatt ctcttg 16
<210> 65
   <211> 17
   <212> DNA
   <213> Glycine max
<400> 65
   accatgcatt cttttga 17
<210> 66
   <211> 22
   <212> DNA
   <213> Glycine max
<400> 66
   tcccttgacg agtctcacac at 22
<210> 67
   <211> 24
   <212> DNA
   <213> Glycine max
<400> 67
   ttgcttcttg ttctcctgaa gttg 24
<210> 68
   <211> 18
   <212> DNA
   <213> Glycine max
<400> 68
   cagacagaaa cgatgata 18
<210> 69
   <211> 17
   <212> DNA
   <213> Glycine max
<400> 69
   cagaaacgat gctattg 17
<210> 70
   <211> 19
   <212> DNA
   <213> Glycine max
<400> 70
   gtggcattgg ccttttggt 19
<210> 71
   <211> 19
   <212> DNA
   <213> Glycine max
<400> 71
   cactgcctct tccctagcc 19
<210> 72
   <211> 18
   <212> DNA
   <213> Glycine max
<400> 72
   catgaaacca aacataat 18
<210> 73
   <211> 18
   <212> DNA
   <213> Glycine max
<400> 73
   catgaaacca gacataat 18
<210> 74
   <211> 29
   <212> DNA
   <213> Glycine max
<400> 74
   gcacgatgaa ttctttgtat gatgtttga 29
<210> 75
   <211> 25
   <212> DNA
   <213> Glycine max
<400> 75
   aaagcataac ttgttgtcgc atcaa 25
<210> 76
   <211> 20
   <212> DNA
   <213> Glycine max
<400> 76
   ttctgtaata cttgattatc 20
<210> 77
   <211> 20
   <212> DNA
   <213> Glycine max
<400> 77
   ttctgtaata catgattatc 20
<210> 78
   <211> 23
   <212> DNA
   <213> Glycine max
<400> 78
   tcccttccag gagaattctg aac 23
<210> 79
   <211> 23
   <212> DNA
   <213> Glycine max
<400> 79
   tgaaaggcag gactctggtt atg 23
<210> 80
   <211> 17
   <212> DNA
   <213> Glycine max
<400> 80
   aaccatcatc cgaatcc 17
<210> 81
   <211> 16
   <212> DNA
   <213> Glycine max
<400> 81
   taaaccatca tccgag 16
<210> 82
   <211> 459
   <212> DNA
   <213> Glycine max
<400> 82
   ttttgatcca aaacaaagct gaaaagaaag gggacaggta tgaagcaatc ttcagcttct 60
   actttggaga ctatggtcac atagcagtgc agggacctta cctgacctat gaggacacat 120
   atttggctgt gactggtggg tctggcatat ttgagggtgt taaaggtcaa gtgaagctgc 180
   gtcagattgt gtatcctttc aagattttgt acacatttta tctaaagggt atcaaggatt 240
   tgcctcagga gcttcttgtc aagactgttg agccaattcc atctgttgaa ccttcccctg 300
   ctgctaaggc ccttgagccc aatgctacca ttgctggctt caccgactaa ttcatcaact 360
   ttttttgtat ttgctttggc ctttgtagta gtatgattta agttactgaa taataataac 420
   aagtggggac tatgatgggt tttgtagtgg tggagtttc 459
<210> 83
   <211> 1102
   <212> DNA
   <213> Glycine max
<400> 83
   ttacaccaga atcatggcca ccaatcaaat acccctatca tcttattaca gaaaagggga 60
   taagagaagg ggaaaaaaaa tctctagaaa ctgaagaatc aaggtttggt tcaaaaactg 120
   tgcagttgcg ttagcatgtg aacaagctca tcactagaag gcctatctgc aggcatatca 180
   gagaggcatg cagcagcaat cctaaccgcc attagcatct catcttcctc accttcttcc 240
   cctaacatgc tcttatctag agcttcgtgc gcctcgcccg cttgctgcaa gtgtctcagc 300
   caacatccca aacttccccc actggctgct tctccaaaga atggatctgt aggatcctta 360
   ccagttaaca aaacacctag tatcatgcca aaactaaaga tgtcactttt gtcagtgtac 420
   ctgctgaaat tgccaaaaaa caccattcaa tctgaatacc atgaagctcc acatatacaa 480
   gaatggaatg aagttaaaat aaacatctta ttggcaatac catacccata tatgcaatgc 540
   catcaatcaa gcaatctttt attgttatta cttattgcta ttgatattga tgcatccaaa 600
   tatacaccac tgaaaaacta gaagtattcc aagttaaatt aggaaaaaaa acctttgtca 660
   attattatta taatttagtt gtggtctcac tcaccaatct aggtttagta gtttgcagca 720
   tgtgaactat aaactatatt attcatttgg accagactta gtgccaactg cctaaggtct 780
   aaaacttgac atcaggaagt aatgggtagt tacaaaaaca aatagcaaag tctttattaa 840
   ataataccaa attcaatcag caaatgatac aatgacaata cagattacag aatagtaatt 900
   tcgtttcaaa acaagagtct ttcattaggg tactacaaag ggggcaaaaa accaatgagg 960
   tgtggagctt ttgaaaaggt aacgaatcaa acaaattccc aatatcaacc acctaattac 1020
   cctattggtg taaaaaagtg aaagaacaga aacagaccaa gttcaaaaca ctagtgaatg 1080
   agtatgattg aaatttgtct ta 1102
<210> 84
   <211> 402
   <212> DNA
   <213> Glycine max
<400> 84
   tcagttacgc aagttttgaa gctgttggtt atgactttaa tgacatggct ctaggtgaac 60
   ttgtggaaag gattttagct tgcttttgtc cagcagagtt ttctgttgct ttgcacattg 120
   acatgcatgg tgagaaactt aataaatttc ccttagacat caaaggatac tactgtggtg 180
   agaggagcac tgaagagctt ggagtaggtg gtgcagttgt gtaccaaaca tttgttcaag 240
   gctgcgatgg tagtgcatct cctaggtcta ttctgaaatg ctgctggagt gaggacgaga 300
   acgaagatga agtgagggag atataagtac ttctggtttt tatgttctgt tgttatttat 360
   ctttactgtc gagtgtgtta ttacctaaat aaataaatat gg 402
<210> 85
   <211> 466
   <212> DNA
   <213> Glycine max
<400> 85
   atcctcaaat acaagtccct gatctcatca tacctaagtt gttaaagaaa atctagccca 60
   ttccatataa atcttattaa taccttactc caggacctga aagacaacct acccaactat 120
   cattaaacaa tttagtctat accacctatg cccaaataga tatagatata tagaaacaaa 180
   ccaggtggaa gaggaataac agagaagtgt ataaaataaa tatgcaactt cagacttgta 240
   tgctcaagcc gcaagcttat atttgaagat gtctatccca caattccaga tccacctaaa 300
   caagaataaa aatccacaaa acattagaag cgaattcgct gctgcaaatt agaatataaa 360
   attcttaaaa ataatcctta ccaaaagatt catcacatta ataatagtat ccatgcccat 420
   gtgagccatc acccccgtaa taagatgaat atgaacctct accata 466
<210> 86
   <211> 489
   <212> DNA
   <213> Glycine max
<400> 86
   atctcaatct gctttgacag atagagagaa ggagaatctt gacaacttag ttgacaagga 60
   gtgtgcaacc acagagggta tgatatcgag ccagctcaag ttgaagtctt ctttgtcatc 120
   caaagcgtct tctcatcaaa gcatggacaa acatgcaatc cttcgacgta tcaggcaacg 180
   caggtttaat aacaaagcca aaaccgctct ggaagctctc ataggcacct cagaagccaa 240
   caataccggc actgcccaag aacagaagtg gcttcaactg ggtgactctt tctcatctcc 300
   ttaactggaa acacaatctc cccccagttt gcaacccatc tataaatacc ctctttagtt 360
   tccttcaccc aacaaagtca tcttttccat acgcttgcat ggctaggaaa ggaaatccaa 420
   cccgatcttt atcggcatgc ttaagaaatc ccactatgta tctattaact gtagtagacc 480
   ttgtcggtt 489
<210> 87
   <211> 794
   <212> DNA
   <213> Glycine max
<400> 87
   ccaaccacgc tgattcttta ggccatcagt caacaaatta ctctggaaac tgctaaggaa      60
   tataaacctt gaaccagaca tgtcccctga agagtagaaa aatatcatgt ttcagcagca     120
   tggttcaaga aaatttgttc atgtaggcaa aattgatttt gaaagaaatt gattatgcaa     180
   aattaatgtt ggttaagatt aattttaaag taatgtgatt tatgtttgaa tattttttat      240
   cgtagcaact cagtataact tttttaattc aatgcaaaaa tattcaaagt tattttgact     300
   caatcaattc tagattcaat atcaattcct catcattgaa acaaatgcca acatgtattt     360
   aaaatcacga tagttgatat ttcaacgtaa ttcaagaaaa ttcaacataa aactaaacac     420
   gcatcactta atgcgtgcta gggtgagcat taaaattgat ttcaaataaa attaattttc     480
   taaaattggt tttggtaaaa attgattttg aagtgacatg agttatgttt gaacgttttt     540
   tattctaaaa gttaataaca aatttcaaat tttttttatc caaagcaaaa gtcatctaaa     600
   agttatttga actcgaaatc aaatttaaac tcaaaatcaa ttccttaatg tgaaattaaa     660
   catgtgtcca tttacctcaa atcatgttat ccgatatttc aacaagactt taaagaatcc     720
   tacgtgaaac caaacatgca cttactagaa taagcagagt gaatagcaga aatacccaga     780
   ttgtaagctg caga                                                       794
<210> 88
   <211> 568
   <212> DNA
   <213> Glycine max
<400> 88
   atgaatagag acaaatccac agaaacaaat aaacagcaca aaagctacaa aagtaaagtc      60
   ttgaggtaaa aaattggcac tagcagaaac aaaatatagt gaaggaacca gatcatgata     120
   aatgctaata aagtgggaaa aaaatcggtt gaataattaa acaaaattaa ttacaaagct     180
   aagtatataa ttcaactgtt agaagaaaaa aagtcgttat gcattcctaa aaggcaaggc     240
   acagtcaacc taaataattt ccctaaatat tttcccctca acccactaca atattttaaa     300
   atagccatga aaatagatta ctagatcaaa attacaaaag taattaatat acaatatcat     360
   tggtttaaca aattacacca cagaagaaaa ataccatcca caaccaagag aagatgacat     420
   aagatattga tatcaaagaa aagtctaggc atataataaa accattaaat cagtacaaac     480
   tacactcaga aataaatagc catcttactg ccaggctaat ataaaaaatg gtcactgaag     540
   tttatgactt tgtgcaactg tgtaaaac                                        568
<210> 89
   <211> 503
   <212> DNA
   <213> Glycine max
<400> 89
   ccgagacgag tgaggaaatc ctcgaggtgg gtcaaggacc cctcgtgctt gcttgggtcg      60
   aacatggagg caacgagagt acaaaatgtt agggacacaa taagctggtt ttcgaggaac     120
   tagtacctcc agcaattcaa taaagaagaa gaagaacaaa gagggaaaga gtgaggtaat     180
   tggaaatttc attcatgcct tgcttgttat ttacattagt atttataaac ttcctagtaa     240
   cagaaagatc ctaggaatat ttgcccataa caaaatttgt acacattgtc ccctaggagc     300
   agacaatatc aaccaaggaa attctagaag gtagcctcag ctggtctcac ccttcttcca     360
   aaaacgtact ctttgaggta agaaggtttt gtaattcttc tttcgaactt agcttcttcc     420
   tgtaccccct catttgcttt tgtgacccct gcccttgcct ctgtttgtaa gctcgtatca    480
   acatgtttcc gatacagatg cag                                             503
<210> 90
   <211> 762
   <212> DNA
   <213> Glycine max
<400> 90
   aacagagttg gctataacaa ggttttggat aatgccaagc ccatgaatga tccagatgga      60
   aggcactttt catcttttac ttacctgagg cttagttcac ttctcatgga acgacaaaac     120
   ttcatagagt ttgaaagatt tgtcaagaga atgcatggta agttgatgaa gaaaatttta     180
   aatgtatctc tagcctctaa ttgatttatg aaaatatcta ttaaggaaag aaaatcatcc     240
   ctttatctgg ccttgattga atgaatgctc acgtgtcatc tgaaaccttc ttactccaca     300
   gtcccacact cagtagggta atcagcaaaa ccctgaagac ccaaatttct ttcagaggca     360
   tggttcagca actgtactaa tatcatattg aactttggat tccattattc gctcccactg     420
   atagtgttct ttgatccacc tagcactagt ctttttatct gattggaatc attttataaa     480
   taattgaaca cgaaaggagt attaatgtca gttttaagag gtgtacttct cttggagaga     540
   aaaacactga aactttaagt agctgcaaaa gttgttgcta tcttgtggga cattgattat     600
   attagggttg atgactgtgt catttctcat tgcaggggaa gctgttcttg atcttcaggt     660
   ataaccggtg ggaaacaaag tcttgttgaa tttgtattta taagtcccct gacaaaatgg     720
   ttgggggtat tctgttatag aataggagaa ataggattgt ta                        762
<210> 91
   <211> 513
   <212> DNA
   <213> Glycine max
<400> 91
   gagaaagaga agtattgcgc atcatgccca aatgtggcca cacttttcat ctttcttgca 60
   ttgatatatg gctgaggaaa caatccacct gtccagtatg ccgtctgccg ttgaaaaact 120
   cttccgaaac gaaacatgtg agacctgtga catttaccat gagccaatcc cttgacgagt 180
   ctcacacatc agacagaaac gatgatattg agagatatgt tgaacctaca cctactgcag 240
   ccagtaactc tttacaacca acttcaggag aacaagaagc aaggcaatga tcttagagaa 300
   ctaaaggggt tgttctgctc aaaaagagaa gaatgtagaa tttctgcttc tatagaggaa 360
   tgcttctaat tatagattgg attcaaattc tttgtctgta atatggcctt catattcact 420
   tggtggtgta aatatgtttc cttttgtagc atatgcgggc caaggttttg gtggaatttc 480
   ttgcataccg atttgaagtt cttttgtcta tgg 513
<210> 92
   <211> 280
   <212> DNA
   <213> Glycine max
<400> 92
   atccaccaac accacttcac caccaaagcc tagtggagct gtaactgttt ccagtggcat 60
   tggccttttg gtggggtctt tctttgtgtc agcaattatg tttggtttca tgggctaggg 120
   aagaggcagt gctgtttttt ttttattctc ctttttatta atatgttatt gttttgctga 180
   gagtttgtgt gaatttcatc atttaatcca gatgtatatc taatcttgat atgtatctcc 240
   ttttaatatt taaaaatgac acatttcttt agctgcttcc 280
<210> 93
   <211> 883
   <212> DNA
   <213> Glycine max
<400> 93
   agatttcgtt tctgggtatc agaaaacacc taggttccga ttagatttct atattttgcc      60
   tagacgtcaa atgcacgatg aattctttgt atgatgtttg aaaattgaag tcttctgtaa     120
   tacttgatta tcaacctcaa attttgatgc gacaacaagt tatgctttgt gaagttgtta     180
   ttgatgattg tatggtgtga tttggtttca ggtttgaaac ttgttactgt ggaccgtcct     240
   tttgctgaga agcactatgc tgatttgtct gccaagcctt tctttagtgg tttggtggat     300
   tacattatct ctggccctgt tgttgccatg atctgggagg gcaagaatgt tgttacaact     360
   ggccgaaaga ttatcggagc cacaaaccca gctcaatctg agcctggaac tatccgtggt     420
   gactttgcca ttgacattgg aaggtataga cttgttttca tcatcgttca agcaagttta     480
   cgttttcaaa tcttttgttt taacacatga atatgaatct gttttcattg ttgttcatgt     540
   ttctaatcta gtttggtgga tgttaggttt tcatttttgt tgtttgattt tcaatttctc     600
   ccgaattttt ttcttattta caacattggt ttccaacgtt ctgtgtttta gagaaaagag     660
   tgttgtgctc aaccaattct gcgaatattg caatatgttc atgacatcat caattaagca     720
   agatgataac actgttgagt acatcataat caaggcagtg gaatccaaac aaatccatgt     780
   tctgtatctt tgtattcatg aaaatcaaaa attgttgttt tagtcaatgt ttatcaacat     840
   tgctcttagt gtggatctag atacttatta tttacaattc tat                       883
<210> 94
   <211> 475
   <212> DNA
   <213> Glycine max
<400> 94
   atgaccaacc taccgcctgt gggtaaagct aaactaagtt ctcacaggtg aggtgagttc      60
   accaaccacg gccgccagaa tttccagatc cccatccgtt ccctgatgct gctttcttgg     120
   gggcagatcc ccagccagag cttccttgtt caccatcact tggaccagca ccaccactcg     180
   cttgtccccc ccagccacca ttgtcactgt tactagctcc acctcctccc cctccccaac     240
   cactactgcc tccaccgctt ccaccaccac caccccaacc accaggaaaa gcttcccttc     300
   caggagaatt ctgaaccttg gcccctggaa aattgcttaa accatcatcc gagtcctttg     360
   tattattaga accccatctg cccccataac cagagtcctg cctttcatta ttggagttgt     420
   ctcctctatt gttataagaa cctcgaccac gaccacgtcc acgccctctt ccacc          475

## Description

### TECHNICAL FIELD

The presently disclosed subject matter relates to markers associated with soybean rust (SBR) resistance and methods of use therefor. More particularly, the presently disclosed subject matter relates to methods for screening, detecting or selecting soybean lines for resistance to SBR, the methods involving genetic marker analysis.

### BACKGROUND

Plant pathogens are known to cause massive damage to important crops, resulting in significant agricultural losses with widespread consequences for both the food supply and other industries that rely on plant materials. As such, there is a long felt need to reduce the incidence and/or impact of agricultural pests on crop production.

Soybean rust (SBR), which is caused by the obligate fungal pathogen *Phakopsora pachyrhizi* H. Sydow & Sydow, was first reported in Japan in 1902. By 1934, the pathogen was reported in several other Asian countries and in Australia. More recently, *P. pachyrhizi* infection has been reported in Africa, and has spread rapidly through the African continent.

In November 2004, *P. pachyrhizi* was first reported in the continental U.S., and the pathogen has now been reported in more than 300 U.S. counties, in Canada, and in Mexico. In 2007, approximately 0.5 million hectares of soybean were sprayed for SBR control in the U.S.

SBR has the potential to cause significant yield losses in the U.S., as indicated by fungicide trials in Georgia and Florida that reported yield losses of 30 to 33% in untreated control plots. In Brazil, the total yield loss in the 2006-2007 growing season due to SBR was estimated to be over U.S. $2.26 billion with an average of 2.3 fungicide applications required per season. Yield losses up to 80% have been reported due to severe outbreaks of SBR, which result in early leaf drop that inhibits pod set. Consistent economic losses in Brazil over the last several years due to severe SBR outbreaks have raised concerns regarding the potential impact of this disease in the United States. Soybean cultivars currently available commercially in the United States are all susceptible to SBR to some degree, and fungicide applications are currently employed to control the disease.

Therefore, soybean rust resistant cultivars are needed to reduce fungicide costs and yield losses due to SBR.

### SUMMARY

The presently disclosed subject matter also provides methods for selecting a soybean rust (SBR) resistant soybean plant. In some embodiments, the methods comprise (a) genotyping one or more soybean plants with respect to one or more single nucleotide polymorphisms (SNPs); and (b) selecting a soybean plant that includes at least one resistance allele associated with the SNPs, thereby selecting an SBR resistant soybean plant.

In some embodiments, the presently disclosed methods comprise (a) isolating one or more nucleic acids from a plurality of soybean plants; (b) detecting in said isolated nucleic acids the presence of one or more marker molecules associated with SBR resistance, wherein said marker molecule is selected from the group consisting of SEQ ID NOs: 1-13; and (c) selecting a soybean plant comprising said one or more marker molecules, thereby selecting an SBR resistant soybean plant.

In some embodiments, the one or more nucleic acid markers are selected from the group consisting of SEQ ID NOs: 1-13, and informative fragments thereof. In some embodiments, the methods and compositions of the presently disclosed subject matter employ a marker molecule mapped within 1, 2, 3, 4, 5, 6, 7, 8, 9, or 10 centiMorgans or less from a marker molecule selected from the group consisting of SEQ ID NOs: 1-13.

In some embodiments, the marker-assisted breeding comprises single nucleotide polymorphism (SNP) analysis. In some embodiments, the methods further comprise screening an introgressed soybean plant, or a cell or tissue thereof, for SBR resistance

In some embodiments of the presently disclosed methods, the at least one resistance allele is associated with an allele having an A at nucleotide 428 of SEQ ID NO: 1; a T at position 895 of SEQ ID NO: 2; a G at position 932 of SEQ ID NO: 2; a T at position 57 of SEQ ID NO: 3; a G at position 213 of SEQ ID NO: 4; a G at position 441 of SEQ ID NO: 4; an A at position 70 of SEQ ID NO: 5; a T at position 348 of SEQ ID NO: 5; an A at position 715 of SEQ ID NO: 6; a C at position 377 of SEQ ID NO: 7; a T at position 100 of SEQ ID NO: 8; a G at position 113 of SEQ ID NO: 8; a T at position 147 of SEQ ID NO: 9; a C at position 205 of SEQ ID NO: 10; an A at position 102 at SEQ ID NO: 11; A T at position 159 of SEQ ID NO: 12; and/or a G at position 357 of SEQ ID NO: 13.

Thus, it is an object of the presently disclosed subject matter to provide methods for detecting the presence of one or more alleles associated with soybean rust resistance in a soybean plant or parts of a soybean plant and methods for selecting a soybean rust (SBR) resistant soybean plant, which object is achieved in whole or in part by the presently disclosed subject matter.

### BRIEF DESCRIPTION OF THE DRAWINGS

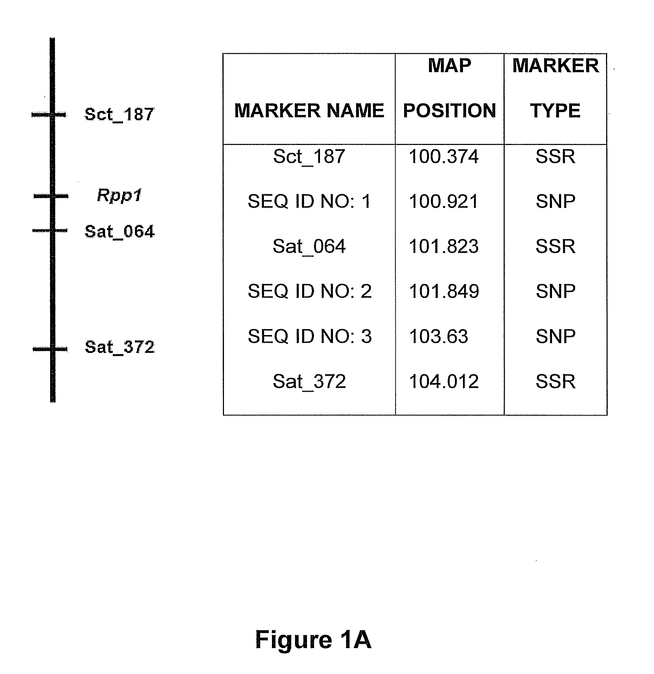

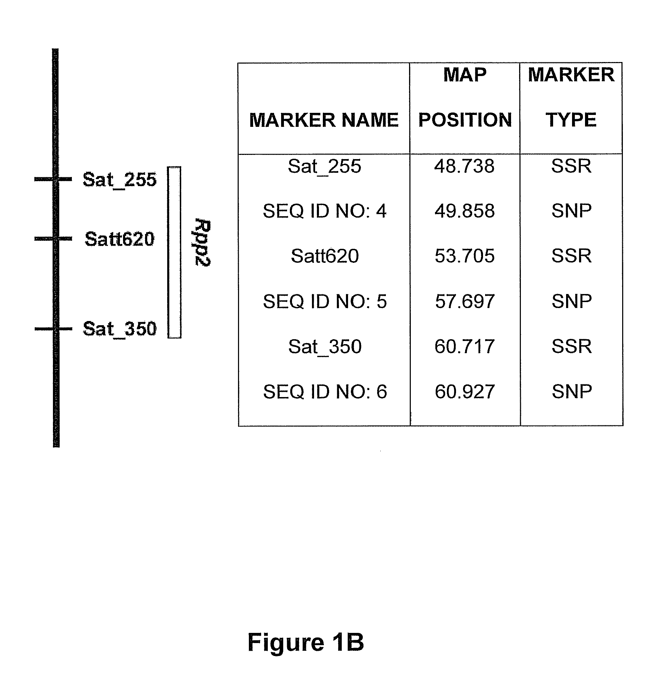

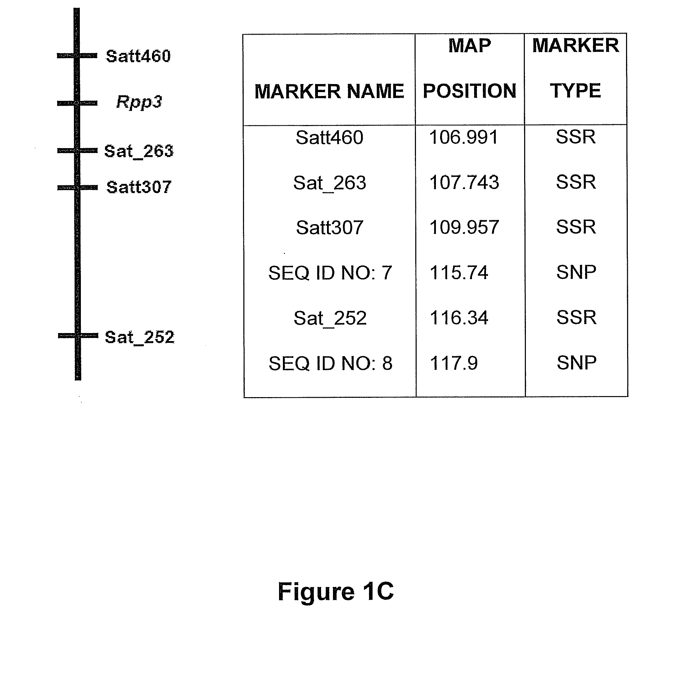

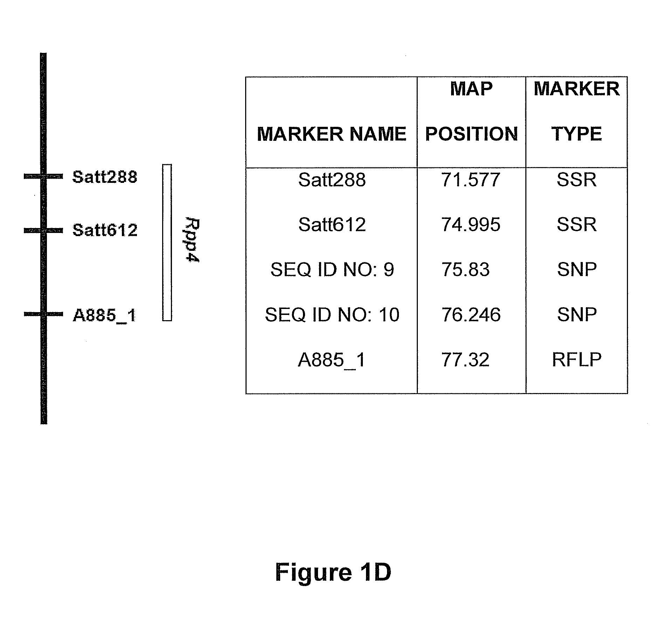

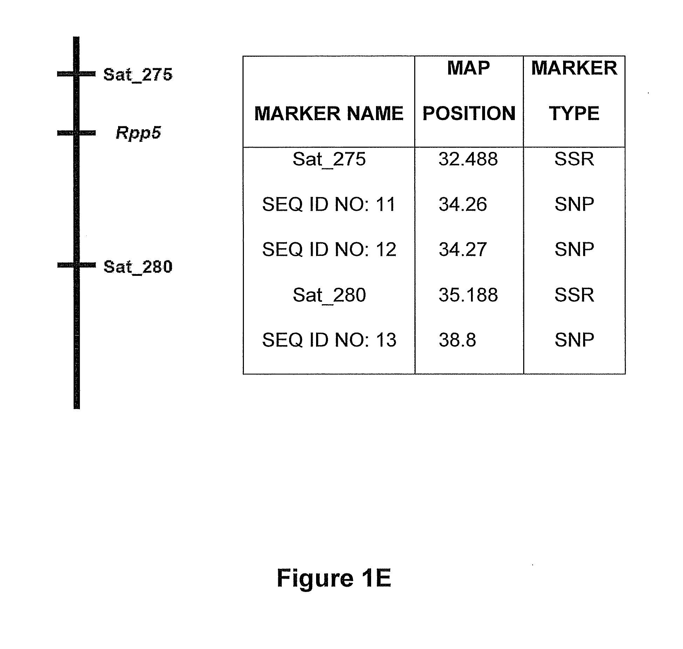

Figures 1A-1E are genetic linkage maps depicting linkage groups and showing relative positions of various markers linked to *Rpp1 - Rpp5*, respectively.

### BRIEF DESCRIPTION OF THE SEQUENCE LISTING

SEQ ID NOs: 1-13 are nucleotide sequences of the soybean genome comprising single nucleotide polymorphisms (SNPs) identified as being associated with the *Rpp1* gene, the *Rpp2* gene, the *Rpp3* gene, the *Rpp4* gene, and/or the *Rpp5* gene as set forth in Table 1.

SEQ ID NOs: 14-81 are nucleotide sequences of oligonucleotide primers that can be employed to amplify and/or otherwise assay a subsequence of the soybean genome that is associated with the *Rpp1-Rpp5* loci as also set forth in Table 1.

**Table 1**

| Amplification and Detection Primers | | | |
|---|---|---|---|
| *Rpp* Gene | Genomic Subsequence SEQ ID NO: | Amplification Primer SEQ ID NOs. | Detection Primers SEQ ID NOs. |
| 1 | 1 | 14 and 15 | 16 and 17 |
| 1 | 2 | 18 and 19 | 20 and 21 |
| 1 | 2 | 22 and 23 | 24 and 25 |
| 1 | 3 | 26 and 27 | 28 and 29 |
| 2 | 4 | 30 and 31 | 32 and 33 |
| 2 | 4 | 34 and 35 | 36 and 37 |
| 2 | 5 | 38 and 39 | 40 and 41 |
| 2 | 5 | 42 and 43 | 44 and 45 |
| 2 | 6 | 46 and 47 | 48 and 49 |
| 3 | 7 | 50 and 51 | 53 and 53 |
| 3 | 8 | 54 and 55 | 56 and 57 |
| 3 | 8 | 58 and 59 | 60 and 61 |
| 4 | 9 | 62 and 63 | 64 and 65 |
| 4 | 10 | 66 and 67 | 68 and 69 |
| 5 | 11 | 70 and 71 | 72 and 73 |
| 5 | 12 | 74 and 75 | 76 and 77 |
| 5 | 13 | 78 and 79 | 80 and 81 |

SEQ ID NOs: 82-94 corresponds to subsequences of the preliminary assembly of the soybean (*Glycine max*) genomic sequence present in the Phytozyme Database, which correspond to SEQ ID NOs: 1-13 as set forth in Table 2.

**Table 2**

| Locations of SEQ ID NOs: 1-13 in the Phytozyme Database | | | |
|---|---|---|---|
| SEQ ID NO: | Relevant Nucleotides | Nucleotide Positions in Phytozyme Database | Percent Identity |
| 1 | 1-459 | 60469363-60469821 | 99.8 |
| 2 | 1-1101 | 60577757-60578858 | 99.1 |
| 3 | 23-424 | 60291869-60292270 | 98.8 |
| 4 | 1-470 | 27963429-27963894 | 99.6 |
| 5 | 1-489 | 30065112-30065600 | 99.4 |
| 6 | 1-794 | 30330727-30331520 | 98.7 |
| 7 | 1-568 | 46665732-46666299 | 99.3 |
| 8 | 1-503 | 41676695-41677197 | 98.8 |
| 9 | 1-769 | 56338383-56339144 | 97.9 |
| 10 | 1-513 | 55961511-55962023 | 99.6 |
| 11 | 1-281 | 33395967-33396246 | 99.3 |
| 12 | 36-916 | 33200392-33201274 | 97.4 |
| 13 | 5-479 | 35318068-35318542 | 99.4 |

### DETAILED DESCRIPTION

The presently disclosed subject matter relates at least in part to the identification of several SNPs associated with SBR resistance in *Glycine sp.* Thus, provided herein are methods of detecting SBR resistance into soybean germplasm, which employ one or more of the identified SNPs in various approaches.

### I. Definitions

Unless defined otherwise, all technical and scientific terms used herein have the same meaning as commonly understood to one of ordinary skill in the art to which the presently disclosed subject matter belongs.

Following long-standing patent law convention, the articles "a", "an", and "the" refer to "one or more" when used in this application, including in the claims. For example, the phrase "a marker" refers to one or more markers. Similarly, the phrase "at least one", when employed herein to refer to an entity, refers to, for example, 1, 2, 3, 4, 5, 6, 7, 8, 9, 10, 15, 20, 25, 30, 35, 40, 45, 50, 75, 100, or more ofthat entity, including but not limited to whole number values between 1 and 100 and greater than 100.

Unless otherwise indicated, all numbers expressing quantities of ingredients, reaction conditions, and so forth used in the specification and claims are to be understood as being modified in all instances by the term "about". Accordingly, unless indicated to the contrary, the numerical parameters set forth in this specification and attached claims are approximations that can vary depending upon the desired properties sought to be obtained by the presently disclosed subject matter.

As used herein, the term "about," when referring to a value or to an amount of mass, weight, time, volume, concentration or percentage is meant to encompass variations of±20%, ±10%, ±5%, ±1%, ±0.5%, and ±0.1% from the specified amount, as such variations are appropriate to perform the disclosed method.

As used herein, the term "allele" refers to any of one or more alternative forms of a gene, all of which relate to at least one trait or characteristic. In a diploid cell, two alleles of a given gene occupy corresponding loci on a pair of homologous chromosomes, although one of ordinary skill in the art understands that the alleles in any particular individual do not necessarily represent all of the alleles that are present in the species. Since the presently disclosed subject matter relates to SNPs, it is in some instances more accurate to refer to a "haplotype" (*i.e*., an allele of a chromosomal segment) instead of "allele". However, in such instances, the term "allele" should be understood to comprise the term "haplotype".

As used herein, the phrase "associated with" refers to a recognizable and/or assayable relationship between two entities. For example, a trait, locus, QTL, SNP, gene, marker, phenotype, etc. is "associated with resistance" if the presence or absence of the trait, locus, QTL, SNP, gene, marker, phenotype, etc., influences an extent or degree of resistance (e.g., resistance to SBR). An allele associated with resistance to SBR comprises an allele having an A at nucleotide 428 of SEQ ID NO: 1; a T at position 895 of SEQ ID NO: 2; a G at position 932 of SEQ ID NO: 2; a T at position 57 of SEQ ID NO: 3; a G at position 213 of SEQ ID NO: 4; a G at position 441 of SEQ ID NO: 4; an A at position 70 of SEQ ID NO: 5; a T at position 348 of SEQ ID NO: 5; an A at position 715 of SEQ ID NO: 6; a C at position 377 of SEQ ID NO: 7; a T at position 100 of SEQ ID NO: 8; a G at position 113 of SEQ ID NO: 8; a T at position 147 of SEQ ID NO: 9; a C at position 205 of SEQ ID NO: 10; an A at position 102 at SEQ ID NO: 11; A T at position 159 of SEQ ID NO: 12; and/or a G at position 357 of SEQ ID NO: 13.

As used herein, the term "backcross", and grammatical variants thereof, refers to a process in which a breeder crosses a progeny individual back to one of its parents, for example, a first generation hybrid F1 with one of the parental genotypes of the F1 hybrid. A backcross can be performed repeatedly, with a progeny individual of one backcross being itself backcrossed to the same parental genotype.

The term "chromosome" is used herein in its art-recognized meaning of the self-replicating genetic structure in the cellular nucleus containing the cellular DNA and bearing in its nucleotide sequence the linear array of genes.

As used herein, the terms "cultivar" and "variety" refer to a group of similar plants that by structural or genetic features and/or performance can be distinguished from other varieties within the same species.

As used herein, the term "gene" refers to a hereditary unit including a sequence of DNA that occupies a specific location on a chromosome and that contains the genetic instruction for a particular characteristics or trait in an organism.

As used herein, the term "hybrid" in the context of plant breeding refers to a plant that is the offspring of genetically dissimilar parents produced by crossing plants of different lines or breeds or species, including but not limited to the cross between two inbred lines.

As used herein, the term "inbred" refers to a substantially homozygous individual or line.

As used herein, the phrase "informative fragment" refers to a nucleic acid molecule and/or its nucleotide sequence that allows for the proper identification of which allele of an allele set *(e.g.,* an SNP) the nucleic acid molecule and/or the nucleotide sequence corresponds to. For example, whereas the SNP that corresponds to SEQ ID NO: 1 relates to an "A" or a "G" at position 428, an "informative fragment" of SEQ ID NO: 1 would be any sequence that comprises position 428 of SEQ ID NO: 1, thereby allowing the nucleotide that is present in that position to be determined.

As used herein, the terms "introgression", "introgressed", and "introgressing" refer to both a natural and artificial process whereby genomic regions of one species, variety, or cultivar are moved into the genome of another species, variety, or cultivar, by crossing those species. The process can optionally be completed by backcrossing to the recurrent parent.

As used herein, the term "linkage" refers to a phenomenon wherein alleles on the same chromosome tend to be transmitted together more often than expected by chance if their transmission was independent. Thus, two alleles on the same chromosome are said to be "linked" when they segregate from each other in the next generation less than 50% of the time, less than 25% of the time, less than 20% of the time, less than 15% of the time, less than 10% of the time, less than 5% of the time, less than 4% of the time, less than 3% of the time, less than 2% of the time, or less than 1% of the time. Thus, two loci are linked if they are within 50, 25, 20, 15, 10, 5, 4, 3, 2, or 1 centiMorgans (cM) of each other. For example, an SNP is linked to a marker if it is within 50, 25, 20, 15, 10,9, 8, 7, 6, 5, 4, 3, 2, or 1 cM of the marker.

As used herein, the phrase "linkage group" refers to all of the genes or genetic traits that are located on the same chromosome. Within the linkage group, those loci that are close enough together can exhibit linkage in genetic crosses. Since the probability of crossover increases with the physical distance between loci on a chromosome, loci for which the locations are far removed from each other within a linkage group might not exhibit any detectable linkage in direct genetic tests. The term "linkage group" is mostly used to refer to genetic loci that exhibit linked behavior in genetic systems where chromosomal assignments have not yet been made. Thus, the term "linkage group" is synonymous with the physical entity of a chromosome, although one of ordinary skill in the art will understand that a linkage group can also be defined as corresponding to a region of *(i.e.,* less than the entirety) of a given chromosome.

As used herein, the term "locus" refers to a position that a given gene or a regulatory sequence occupies on a chromosome of a given species.

As used herein, the term "marker" refers to an identifiable position on a chromosome the inheritance of which can be monitored. A marker can comprise a known or detectable nucleic acid sequence.

A marker can correspond to an amplification product generated by amplifying a *Glycine sp.* nucleic acid with two oligonucleotide primers, for example, by the polymerase chain reaction (PCR). As used herein, the phrase "corresponds to an amplification product" in the context of a marker refers to a marker that has a nucleotide sequence that is the same (allowing for mutations introduced by the amplification reaction itself) as an amplification product that is generated by amplifying *Glycine sp.* genomic DNA with a particular set of primers. The amplifying can be by PCR, and the primers are PCR primers that are designed to hybridize to opposite strands of the *Glycine sp.* genomic DNA in order to amplify a *Glycine sp.* genomic DNA sequence present between the sequences to which the PCR primers hybridize in the *Glycine sp.* genomic DNA. A marker that "corresponds to" an amplified fragment can be a marker that has the same sequence of one of the strands of the amplified fragment.

As used herein, the term "soybean" refers to a plant, or a part thereof, of the genus *Glycine* including, but not limited to *Glycine max.*

As used herein, the phrase "soybean-specific DNA sequence" refers to a polynucleotide sequence having a nucleotide sequence homology of more than 50%, more than 60%, more than 70%, more than 80%, more than 85%, more than 90%, more than 92%, more than 95%, more than 96%, more than 97%, more than 98%, and more than 99% with a sequence of the genome of the species *Glycine* that shows the greatest similarity to it. In the case of markers for any of the *Rpp* genes, a "soybean-specific DNA sequence" can comprise a part of the DNA sequence of a soybean genome that flanks and/or is a part of an *Rpp* gene sequence.

As used herein, the phrase "molecular marker" refers to an indicator that is used in methods for visualizing differences in characteristics of nucleic acid sequences. Examples of such indicators are restriction fragment length polymorphism (RFLP) markers, amplified fragment length polymorphism (AFLP) markers, single nucleotide polymorphisms (SNPs), insertion and deletion mutations (INDEL), microsatellite markers (SSRs), sequence-characterized amplified regions (SCARs), cleaved amplified polymorphic sequence (CAPS) markers or isozyme markers or combinations of the markers described herein which defines a specific genetic and chromosomal location. A molecular marker "linked to" or "associated with" an *Rpp* gene as defined herein can thus refer to SNPs, insertion mutations, as well as more usual AFLP markers or any other type of marker used in the field.

As used herein, the phrase "nucleotide sequence homology" refers to the presence of homology between two polynucleotides. Polynucleotides have "homologous" sequences if the sequence of nucleotides in the two sequences is the same when aligned for maximum correspondence. The "percentage of sequence homology" for polynucleotides, such as 50, 60, 70, 80, 90, 95, 98, 99 or 100 percent sequence homology, can be determined by comparing two optimally aligned sequences over a comparison window (e.g., about 20-200 contiguous nucleotides), wherein the portion of the polynucleotide sequence in the comparison window can include additions or deletions (*i.e*., gaps) as compared to a reference sequence for optimal alignment of the two sequences. Optimal alignment of sequences for comparison can be conducted by computerized implementations of known algorithms, or by visual inspection. Readily available sequence comparison and multiple sequence alignment algorithms are, respectively, the Basic Local Alignment Search Tool (BLAST; Altschul et al. (1990) J Mol Biol 215:403-10; Altschul et al. (1997) Nucleic Acids Res 25:3389-3402) and ClustalX (Chenna et al. (2003) Nucleic Acids Res 31:3497-3500) programs, both available on the Internet. Other suitable programs include, but are not limited to, GAP, BestFit, PlotSimilarity, and FASTA, which are part of the Accelrys GCG Package available from Accelrys Software, Inc. of San Diego, California, United States of America.

As used herein, the term "offspring" plant refers to any plant resulting as progeny from a vegetative or sexual reproduction from one or more parent plants or descendants thereof. For instance, an offspring plant can be obtained by cloning or selfing of a parent plant or by crossing two parent plants and include selfings as well as the F1 or F2 or still further generations. An F1 is a first-generation offspring produced from parents at least one of which is used for the first time as donor of a trait, while offspring of second generation (F2) or subsequent generations (F3, F4, and the like) are specimens produced from selfings or crossings of F1s, F2s and the like. An F1 can thus be a hybrid resulting from a cross between two true breeding parents (the phrase "true-breeding" refers to an individual that is homozygous for one or more traits), while an F2 can be an offspring resulting from self-pollination of the F1 hybrids.

As used herein, the term "phenotype" refers to a detectable characteristic of a cell or organism, which characteristics are at least partially a manifestation of gene expression.

As used herein, the phrase "plant part" refers to a part of a plant, including single cells and cell tissues such as plant cells that are intact in plants, cell clumps, and tissue cultures from which plants can be regenerated. Examples of plant parts include, but are not limited to, single cells and tissues from pollen, ovules, leaves, embryos, roots, root tips, anthers, flowers, fruits, stems, shoots, and seeds; as well as scions, rootstocks, protoplasts, calli, and the like.

As used herein, the term "population" refers to a genetically heterogeneous collection of plants sharing a common genetic derivation.

As used herein, the term "primer" refers to an oligonucleotide which is capable of annealing to a nucleic acid target and serving as a point of initiation of DNA synthesis when placed under conditions in which synthesis of a primer extension product is induced *(e.g.,* in the presence of nucleotides and an agent for polymerization such as DNA polymerase and at a suitable temperature and pH). A primer (an extension primer or an amplification primer) can be single stranded for maximum efficiency in extension and/or amplification. The primer can be an oligodeoxyribonucleotide. A primer is typically sufficiently long to prime the synthesis of extension and/or amplification products in the presence of the agent for polymerization. The minimum lengths of the primers can depend on many factors, including, but not limited to temperature and composition (A/T vs. G/C content) of the primer.

In the context of amplification primers, these are typically provided as a pair of bi-directional primers consisting of one forward and one reverse primer or provided as a pair of forward primers as commonly used in the art of DNA amplification such as in PCR amplification.

As such, it will be understood that the term "primer", as used herein, can refer to more than one primer, particularly in the case where there is some ambiguity in the information regarding the terminal sequence(s) of the target region to be amplified. Hence, a "primer" can include a collection of primer oligonucleotides containing sequences representing the possible variations in the sequence or includes nucleotides which allow a typical base pairing.

Primers can be prepared by any suitable method. Methods for preparing oligonucleotides of specific sequence are known in the art, and include, for example, cloning and restriction of appropriate sequences and direct chemical synthesis. Chemical synthesis methods can include, for example, the phospho di- or tri-ester method, the diethylphosphoramidate method and the solid support method disclosed in U.S. Patent No. 4,458,066.

Primers can be labeled, if desired, by incorporating detectable moieties by for instance spectroscopic, fluorescence, photochemical, biochemical, immunochemical, or chemical moieties.

The PCR method is well described in handbooks and known to the skilled person. After amplification by PCR, target polynucleotides can be detected by hybridization with a probe polynucleotide which forms a stable hybrid with that of the target sequence under stringent to moderately stringent hybridization and wash conditions. If it is expected that the probes are essentially completely complementary (*i.e*., about 99% or greater) to the target sequence, stringent conditions can be used. If some mismatching is expected, for example if variant strains are expected with the result that the probe will not be completely complementary, the stringency of hybridization can be reduced. Conditions can be chosen to rule out non-specific/adventitious binding. Conditions that affect hybridization, and that select against non-specific binding are known in the art, and are described in, for example, Sambrook & Russell (2001). Molecular Cloning: A Laboratory Manual, Third Edition, Cold Spring Harbor Laboratory Press, Cold Spring Harbor, New York, United States of America. Generally, lower salt concentration and higher temperature hybridization and/or washes increase the stringency of hybridization conditions.

Continuing, the term "probe" refers to a single-stranded oligonucleotide sequence that will form a hydrogen-bonded duplex with a complementary sequence in a target nucleic acid sequence analyte or its cDNA derivative.

As used herein, the terms "*Rpp1*"*,* "*Rpp2*", "*Rpp3*", "*Rpp4*", and "*Rpp5*" refer to loci that have been associated with SBR resistance as defined by the markers defined herein. For the purposes of the instant disclosure, these loci are said to be present on *Glycine* linkage groups G, J, C2, G, and N, and linked to the markers depicted in Figures 1A-1E, respectively.

As used herein, the term "quantitative trait locus" (QTL; plural quantitative trait loci; QTLs) refers to a genetic locus (or loci) that controls to some degree a numerically representable trait that can be continuously distributed. As such, the term QTL is used herein in its art-recognized meaning to refer to a chromosomal region containing alleles *(e.g.,* in the form of genes or regulatory sequences) associated with the expression of a quantitative phenotypic trait. Thus, a QTL "associated with" resistance to SBR refers to one or more regions located on one or more chromosomes and/or in one or more linkage groups that includes at least one gene the expression of which influences a level of resistance and/or at least one regulatory region that controls the expression of one or more genes involved in resistance to SBR. QTLs can be defined by indicating their genetic location in the genome of a specific *Glycine sp.* accession using one or more molecular genomic markers. One or more markers, in turn, indicate a specific locus. Distances between loci are usually measured by the frequency of crossovers between loci on the same chromosome. The farther apart two loci are, the more likely that a crossover will occur between them. Conversely, if two loci are close together, a crossover is less likely to occur between them. Typically, one centiMorgan (cM) is equal to 1% recombination between loci. When a QTL can be indicated by multiple markers, the genetic distance between the end-point markers is indicative of the size of the QTL.

As used herein, the term "regenerate", and grammatical variants thereof, refers to the production of a plant from tissue culture.

As used herein, the term "resistant" and "resistance" encompass both partial and full resistance to infection (*e*.*g*., infection by a pathogen that causes SBR). A susceptible plant can either be non-resistant or have lower levels of resistance to infection relative to a resistant plant. The term is used to include such separately identifiable forms of resistance as "full resistance", "immunity", "intermediate resistance", "partial resistance", and "hypersensitivity".

As used herein, the phrase "stringent hybridization conditions" refers to conditions under which a polynucleotide hybridizes to its target subsequence, typically in a complex mixture of nucleic acids, but to essentially no other sequences. Stringent conditions are sequence-dependent and can be different under different circumstances. Exemplary guidelines for the hybridization of nucleic acids can be found in Tijssen (1993) in Laboratory Techniques in Biochemistry and Molecular Biology, Elsevier, New York, New York, United States of America; Ausubel et al. (1999) Short Protocols in Molecular Biology Wiley, New York, New York, United States of America; and Sambrook & Russell, 2001 (*supra*). Generally, stringent conditions are selected to be about 5-10°C lower than the thermal melting point (Tm) for the specific sequence at a defined ionic strength pH. The Tm is the temperature (under defined ionic strength, pH, and nucleic acid concentration) at which 50% of the probes complementary to the target hybridize to the target sequence at equilibrium (as the target sequences are present in excess, at Tm, 50% of the probes are occupied at equilibrium). Stringent conditions are those in which the salt concentration is less than about 1.0 M sodium ion, typically about 0.01 to 1.0 M sodium ion concentration (or other salts) at pH 7.0 to 8.3 and the temperature is at least about 30°C for short probes *(e.g.,* 10 to 50 nucleotides) and at least about 60°C for long probes (e.g., greater than 50 nucleotides). Stringent conditions can also be achieved with the addition of destabilizing agents such as formamide. Exemplary stringent hybridization conditions include: 50% formamide, 5x SSC, and 1% SDS, incubating at 42°C; or 5x SSC, 1% SDS, incubating at 65°C; with one or more washes in 0.2x SSC and 0.1% SDS at 65°C. For PCR, a temperature of about 36°C is typical for low stringency amplification, although annealing temperatures can vary between about 32°C and 48°C (or higher) depending on primer length.

As used herein, the term "susceptible" refers to a plant having no resistance to the disease resulting in the plant being affected by the disease, resulting in disease symptoms. The term "susceptible" is therefore equivalent to "non-resistant". Alternatively, the term "susceptible" can be employed in a relative context, in which one plant is considered "susceptible" because it is less resistant to a particular pathogen than is a second plant (which in the context of these terms in a relative usage, would be referred to as the "resistant" plant").

### II. General Considerations

Previous studies on host resistance to *P. pachyrhizi* have resulted in the identification of the four major resistance genes, *Rpp1*, *Rpp2*, *Rpp3,* and *Rpp4,* in soybean accessions PI 200492, PI 230970, PI 462312, and PI 459025B, respectively. Three of these genes (*Rpp2*, *Rpp3,* and *Rpp4)* confer a resistant reddish-brown (RB) colored lesion as opposed to the susceptible tan (TAN) colored lesion. *Rpp1*, on the other hand, confers resistance to some rust isolates. These four genes have been mapped on linkage groups (LGs) G, J, C2, and G, respectively. An RB lesion type resistance gene *Rpp?*(Hyuuga) from the Japanese cultivar "Hyuuga" has been mapped by to the same region on LG C2 as *Rpp3.* A fifth gene, *Rpp5*, was recently identified from PI200456 and mapped on LG N. With the availability of the 7X sequence coverage of the soybean genome made possible by efforts of the U. S. Department of Energy-Joint Genome Institute (DOE-JGI), *Rpp1* has been fine mapped to a 23 kb region on scaffold 21 of LG G, and several markers close to this gene have been identified (Hyten *et al*. (2008) Theor Appl Genet 116:945-952).

Resistance conferred by *Rpp1*, *Rpp2*, *Rpp3, Rpp4,* and *Rpp5* can be race-specific, and can be overcome by various *P. pachyrhizi* isolates. For example, resistance in soybean lines carrying either *Rpp1* or *Rpp3* genes failed in Brazil within two years of the establishment of the disease.

The presently disclosed subject matter provides in some embodiments methods for identifying soybean plants that carry desirable resistance genes.

### III. Plant Breeding

The present description discloses better models for marker-assisted breeding (MAB). Also disclosed are methods of plant breeding and to methods to select plants, in particular soybean plants, particularly cultivated soybean plants as breeder plants for use in breeding programs or cultivated soybean plants for having desired genotypic or potential phenotypic properties, in particular related to producing valuable soybeans, also referred to herein as commercially valuable plants. Herein, a cultivated plant is defined as a plant being purposely selected or having been derived from a plant having been purposely selected in agricultural or horticultural practice for having desired genotypic or potential phenotypic properties, for example a plant obtained by inbreeding.

The presently disclosed subject matter thus also provides methods for selecting a plant of the genus *Glycine* exhibiting resistance towards SBR comprising detecting in the plant the presence of one or more resistance alleles as defined herein. In an exemplary embodiment of the presently disclosed methods for selecting such a plant, the method comprises providing a sample of genomic DNA from a soybean plant; and (b) detecting in the sample of genomic DNA at least one molecular marker associated with resistance to SBR. In some embodiments, the detecting can comprise detecting one or more SNPs that are associated with resistance to SBR.

The providing of a sample of genomic DNA from a soybean plant can be performed by standard DNA isolation methods well known in the art.

The detecting of a molecular marker can comprise the use of one or more sets of primer pairs that can be used to produce one or more amplification products that are suitable markers for one of the SNPs. Such a set of primers can comprise nucleotide sequences as set forth in SEQ ID NOs: 14-81.

The detecting of a molecular marker can comprise the use of a nucleic acid probe having a base sequence that is substantially complementary to the nucleic acid sequence defining the molecular marker and which nucleic acid probe specifically hybridizes under stringent conditions with a nucleic acid sequence defining the molecular marker. A suitable nucleic acid probe can for instance be a single strand of the amplification product corresponding to the marker. The detecting of a molecular marker can be designed to discriminate whether a particular allele of an SNP is present or absent in a particular plant.

The presently disclosed methods can also include detecting an amplified DNA fragment associated with the presence of a particular allele of an SNP. The amplified fragment associated with a particular allele of an SNP has a predicted length or nucleic acid sequence, and detecting an amplified DNA fragment having the predicted length or the predicted nucleic acid sequence is performed such that the amplified DNA fragment has a length that corresponds (plus or minus a few bases; *e.g.,* a length of one, two or three bases more or less) to the expected length as based on a similar reaction with the same primers with the DNA from the plant in which the marker was first detected or the nucleic acid sequence that corresponds (*i.e*., has a homology of more than 80%, more than 90%, more than 95%, more than 97%, and more than 99%) to the expected sequence as based on the sequence of the marker associated with that SNP in the plant in which the marker was first detected. Upon a review of the instant disclosure, one of ordinary skill in the art would appreciate that markers (e.g., SNP alleles) that are absent in resistant plants, while they were present in the susceptible parent(s) (so-called trans-markers), can also be useful in assays for detecting resistance among offspring plants.

The detecting of an amplified DNA fragment having the predicted length or the predicted nucleic acid sequence can be performed by any of a number or techniques, including but not limited to standard gel-electrophoresis techniques or by using automated DNA sequencers. The methods are not described here in detail as they are well known to the skilled person, although exemplary approaches are set forth in the EXAMPLES.

### IV. Molecular Markers and SNPs

Molecular markers are used for the visualization of differences in nucleic acid sequences. This visualization can be due to DNA-DNA hybridization techniques after digestion with a restriction enzyme (e.g., an RFLP) and/or due to techniques using the polymerase chain reaction (e.g., STS, SSR/microsatellites, AFLP, and the like). All differences between two parental genotypes segregate in a mapping population based on the cross of these parental genotypes. The segregation ofthe different markers can be compared and recombination frequencies can be calculated. Methods for mapping markers in plants are disclosed in, for example, Glick & Thompson (1993) Methods in Plant Molecular Biology and Biotechnology, CRC Press, Boca Raton, Florida, United States of America; Zietkiewicz et al. (1994) Genomics 20:176-183.

The recombination frequencies of molecular markers on different chromosomes and/or in different linkage groups are generally 50%. Between molecular markers located on the same chromosome or in the same linkage group, the recombination frequency generally depends on the distance between the markers. A low recombination frequency typically corresponds to a low genetic distance between markers on a chromosome. Comparing all recombination frequencies results in the most logical order of the molecular markers on the chromosomes or in the linkage groups. This most logical order can be depicted in a linkage map. A group of adjacent or contiguous markers on the linkage map that is associated with an increased level of resistance to a disease; *e.g.,* to a reduced incidence of acquiring the disease upon infectious contact with the disease agent and/or a reduced lesion growth rate upon establishment of infection, can provide the position of a locus associated with resistance to that disease.

The markers disclosed herein can be used in various aspects of the presently disclosed subject matter as set forth hereinbelow. Other than the genetic unit "gene", on which the phenotypic expression depends on a large number of factors that cannot be predicted, the genetic unit "QTL" denotes a region of the genome that is directly related to a phenotypic quantifiable trait.

The markers provided by the presently disclosed subject matter can be used for detecting the presence of one or more SBR resistance alleles of the presently disclosed subject matter in a suspected SBR-resistant soybean plant, and can therefore be used in methods involving selection of SBR resistant soybean plants. In some embodiments, detecting the presence of a particular allele of an SNP of the presently disclosed subject matter is performed with at least one of the markers for the resistance loci defined herein. The presently disclosed subject matter therefore relates in another aspect to a method for detecting the presence of a particular allele associated with SBR resistance, comprising detecting the presence of a nucleic acid sequence of the SNP in a suspected SBR-resistant soybean plant, which presence can be detected by the use of the disclosed markers and oligonucleotides.

The nucleotide sequence of an SNP of the presently disclosed subject matter can for instance be resolved by determining the nucleotide sequence of one or more markers associated with the SNP and designing internal primers for the marker sequences that can be used to determine which allele of the SNP is present in the plant.

In such methods for detecting the presence of an SNP in a suspected SBR-resistant soybean plant, the method can also comprise providing a oligonucleotide or polynucleotide capable of hybridizing under stringent hybridization conditions to a particular nucleic acid sequence of an SNP, selected from the SNPs disclosed herein, contacting the oligonucleotide or polynucleotide with genomic nucleic acid (or a fragment thereof, including, but not limited to a restriction fragment thereof) of a suspected SBR-resistant soybean plant, and determining the presence of specific hybridization of the oligonucleotide or polynucleotide to the genomic nucleic acid (or the fragment thereof).

The method can be performed on a nucleic acid sample obtained from the suspected SBR-resistant soybean plant, although *in situ* hybridization methods can also be employed. Alternatively, one of ordinary skill in the art can design specific hybridization probes or oligonucleotides capable of hybridizing under stringent hybridization conditions to the nucleic acid sequence of the allele associated with SBR resistance and can use such hybridization probes in methods for detecting the presence of an SNP allele disclosed herein in a suspected SBR-resistant soybean plant.

### V. Production of SBR-resistant Soybean Plants

Also disclosed are methods for producing an SBR-resistant soybean plant comprising performing a method for detecting the presence of an allele associated with resistance to SBR in a donor soybean plant according to the presently disclosed subject matter as described above, and transferring a nucleic acid sequence comprising at least one allele thus detected, or an SBR resistance-conferring part thereof, from the donor plant to an SBR-susceptible recipient soybean plant. The transfer of the nucleic acid sequence can be performed by any of the methods disclosed herein.

Such a method can comprise the transfer by introgression of the nucleic acid sequence from an SBR-resistant donor soybean plant into an SBR-susceptible recipient soybean plant by crossing the plants. This transfer can thus suitably be accomplished by using traditional breeding techniques. SBR-resistance loci can be introgressed into commercial soybean varieties using marker-assisted selection (MAS) or marker-assisted breeding (MAB). MAS and MAB involves the use of one or more of the molecular markers for the identification and selection of those offspring plants that contain one or more of the genes that encode for the desired trait. Such identification and selection is based on selection of SNP alleles of the presently disclosed subject matter or markers associated therewith. MAB can also be used to develop near-isogenic lines (NIL) harboring the QTL of interest, allowing a more detailed study of each QTL effect and is also an effective method for development of backcross inbred line (BIL) populations. Soybean plants can advantageously derive a majority of their traits from the recipient plant, and derive SBR resistance from the donor plant.

As discussed hereinabove, traditional breeding techniques can be used to introgress a nucleic acid sequence encoding SBR resistance into an SBR-susceptible recipient soybean plant. A donor soybean plant that exhibits resistance to SBR and comprising a nucleic acid sequence encoding SBR resistance can be crossed with an SBR-susceptible recipient soybean plant that can exhibit commercially desirable characteristics, such as, but not limited to, disease resistance, insect resistance, valuable nutritional characteristics, and the like. The resulting plant population (representing the F1 hybrids) is then self-pollinated and set seeds (F2 seeds). The F2 plants grown from the F2 seeds are then screened for resistance to SBR. The population can be screened in a number of different ways.

First, the population can be screened using a traditional disease screen. Such disease screens are known in the art. A quantitative bioassay can be used. Second, marker-assisted breeding can be performed using one or more of the herein-described molecular markers to identify those progeny that comprise a nucleic acid sequence encoding for SBR resistance. Other methods, referred to hereinabove by methods for detecting the presence of an allele associated with SBR resistance, can be used. Also, marker-assisted breeding can be used to confirm the results obtained from the quantitative bioassays, and therefore, several methods can also be used in combination.

Inbred SBR-resistant soybean plant lines can be developed using the techniques of recurrent selection and backcrossing, selfing, and/or dihaploids, or any other technique used to make parental lines. In a method of recurrent selection and backcrossing, SBR resistance can be introgressed into a target recipient plant (the recurrent parent) by crossing the recurrent parent with a first donor plant, which differs from the recurrent parent and is referred to herein as the "non-recurrent parent". The recurrent parent is a plant that is non-resistant or has a low level of resistance to SBR and possesses commercially desirable characteristics, such as, but not limited to (additional) disease resistance, insect resistance, valuable nutritional characteristics, and the like. The non-recurrent parent exhibits SBR resistance and comprises a nucleic acid sequence that encodes SBR resistance. The non-recurrent parent can be any plant variety or inbred line that is cross-fertile with the recurrent parent.

The progeny resulting from a cross between the recurrent parent and non-recurrent parent are backcrossed to the recurrent parent. The resulting plant population is then screened for the desired characteristics, which screening can occur in a number of different ways. For instance, the population can be screened using phenotypic pathology screens or quantitative bioassays as known in the art. Alternatively, instead of using bioassays, MAB can be performed using one or more of the hereinbefore described molecular markers, hybridization probes, or polynucleotides to identify those progeny that comprise a nucleic acid sequence encoding SBR resistance. Also, MAB can be used to confirm the results obtained from the quantitative bioassays. The markers defined herein are suitable to select proper offspring plants by genotypic screening.

Following screening, the F1 hybrid plants that exhibit an SBR-resistant phenotype or, genotype and thus comprise the requisite nucleic acid sequence encoding SBR resistance, are then selected and backcrossed to the recurrent parent for a number of generations in order to allow for the soybean plant to become increasingly inbred. This process can be performed for two, three, four, five, six, seven, eight, or more generations. In principle, the progeny resulting from the process of crossing the recurrent parent with the SBR-resistant non-recurrent parent are heterozygous for one or more genes that encode SBR resistance.

In general is disclosed a method of introducing a desired trait into a hybrid soybean variety can comprise:
(a) crossing an inbred soybean parent with another soybean plant that comprises one or more desired traits, to produce F1 progeny plants, wherein the desired trait is SBR resistance;
(b) selecting the F1 progeny plants that have the desired trait to produce selected F1 progeny plants, using molecular markers as defined herein;
(c) backcrossing the selected progeny plants with the inbred soybean parent plant to produce backcross progeny plants;
(d) selecting for backcross progeny plants that have the desired trait and morphological and physiological characteristics of the inbred soybean parent plant, wherein the selection comprises the isolation of genomic DNA and testing the DNA for the presence of at least one molecular marker for SBR resistance, as described herein;
(e) repeating steps (c) and (d) two or more times in succession to produce selected third or higher backcross progeny plants;
(f) optionally selfing selected backcross progeny in order to identify homozygous plants; and
(g) crossing at least one of the backcross progeny or selfed plants with another soybean parent plant to generate a hybrid soybean variety with the desired trait and all of the morphological and physiological characteristics of hybrid soybean variety when grown in the same environmental conditions.

As indicated, the last backcross generation can be selfed in order to provide for homozygous pure breeding (inbred) progeny for SBR resistance. Thus, the result of recurrent selection, backcrossing, and selfing is the production of lines that are genetically homogenous for the genes associated with SBR resistance, as well as for other genes associated with traits of commercial interest.

### VI. SBR-resistant Soybean Plants and Seeds

The development of a hybrid soybean variety in a soybean plant breeding program can involve three steps: (1) the selection of plants from various germplasm pools for initial breeding crosses; (2) the selfing of the selected plants from the breeding crosses for several generations to produce a series of inbred lines, which, individually breed true and are highly uniform; and (3) crossing a selected variety with an different variety to produce the hybrid progeny (F1). After a sufficient amount of inbreeding successive filial generations will merely serve to increase seed of the developed inbred. An inbred line can comprise homozygous alleles at about 95% or more of its loci.

The SBR-resistant soybean plants, or part thereof, can be heterozygous or homozygous for the resistance traits (in some embodiments, homozygous).

The SBR-resistant soybean lines disclosed herein can be used in additional crossings to create SBR-resistant plants. For example, a first SBR-resistant soybean plant can be crossed with a second soybean plant possessing commercially desirable traits such as, but not limited to, disease resistance, insect resistance, desirable nutritional characteristics, and the like. This second soybean line can itself be SBR-resistant. This line can be heterozygous or homozygous for one or more of the disclosed SBR resistance loci, in order for one or more of these traits to be expressed in the hybrid offspring plants.

Also disclosed is a method of producing seeds that can be grown into SBR-resistant soybean plants. The method can comprise providing a SBR-resistant soybean plant, crossing the SBR-resistant plant with another soybean plant, and collecting seeds resulting from the cross, which when planted, produce SBR-resistant soybean plants.

The method can comprise providing a SBR-resistant soybean plant, crossing the SBR-resistant plant with a soybean plant, collecting seeds resulting from the cross, regenerating the seeds into plants, selecting SBR-resistant plants by any of the methods described herein, self-pollinating the selected plants for a sufficient number of generations to obtain plants that are fixed for an allele associated with SBR-resistance in the plants, backcrossing the plants thus produced with soybean plants having desirable phenotypic traits for a sufficient number of generations to obtain soybean plants that are SBR-resistant and have desirable phenotypic traits, and collecting the seeds produced from the plants resulting from the last backcross, which when planted, produce soybean plants which are SBR-resistant.

### VIII. Other Applications

With the use of these SNPs for breeding new soybean lines, a system for developing germplasm that has more than one mode of action against the fungus is made possible. The use of this dual mode of action will assist in inhibiting the fungus from developing resistance. Additionally, a regime for inhibiting fungal resistance can include a number of different modes of action. The different modes of action in an inhibiting fungus method could be through germplasm or seed treatments or chemical applications. A system for inhibiting fungal resistance includes germplasm with one or more vertical resistant traits, and/or germplasm with one or more horizontal tolerance traits with the possible options of including a seed treatment that is active against the fungus, and/ or an antifungal spray. Antifungal sprays or treatments can include known antifungal compounds for this fungus but can also include glufosinate or glyphosate sprays, separate or in combination which also have an antifungal affect.

The regime for inhibiting antifungal resistance is very important for the continued effectiveness of germplasm resistance and chemical activity. There are areas in which soybeans are presently produced which have fungal isolates that are no longer negatively affected by at least two of the known *Rpp* resistance alleles. The ability of these fungal isolates to evolve so quickly could render entire soybean growing areas unprofitable unless soybean seeds which are protected by the SNP selected traits for a dual mode of action plus the optional seed treatment, or chemical applications is implemented.

### EXAMPLES

The following Examples provide illustrative embodiments. In light ofthe present disclosure and the general level of skill in the art, those of skill will appreciate that the following Examples are intended to be exemplary only and that numerous changes, modifications, and alterations can be employed without departing from the scope ofthe presently claimed subject matter.

### EXAMPLE 1

### SNP Analysis

There are SSR markers which are associated with soybean rust resistant qualitative and quantitative genes *Rpp1*, *Rpp2*, *Rpp3, Rpp4,* and *Rpp5*. This SSR information was employed to identify SNP markers that map to the regions of qualitative genes and quantitative trait loci (QTL) regions defined for each of the *Rpp1*, *Rpp2*, *Rpp3*, *Rpp4,* and *Rpp5* genes. The identified SNPs were validated on soybean rust resistant and susceptible lines. Analyses indicated that these SNPs mapped more closely and showed better associations to the *Rpp* gene than did the SSRs. The information of validated SNPs for soybean rust is new and is used for appropriate breeding programs.

### EXAMPLE 2

### SNP Genotyping Data

Molecular markers were identified for the *Rpp1*, *Rpp2*, *Rpp3, Rpp4,* and *Rpp5* genes, and the resistance gene first identified in the Japanese cultivar, Hyuuga. Hyten *et al*., 2007 used PI200492 to map *Rpp1* to linkage group (LG) G between the SSR markers Sct_187 and Sat_064. *Rpp2* was mapped in PI230970 to the region on LG J between Sat_255 and Satt620. *Rpp4* was mapped in PI459025 on LG G between SSR Satt288 and RFLP marker A885_1 (Silva *et al*., 2008, *supra*). The cultivar Hyuuga (PI506764) was once thought to contain *Rpp3* (the resistance gene contained in PIs 462312 and 459025B), but it is now believed that there are 2 separate rust resistance genes located near each other on LG C2, flanked by SSRs Satt460 and Sat_263. *Rpp5* was recently identified in several lines (PI200487, PI200526, PI471904, PI200456) by Garcia *et al*., 2008, and mapped to LG N in a region flanked by the SSRs Sat_275 and Sat_280. The approximate positions of these genes and various markers are depicted in Figures 1A-1E.

The soybean linkage map developed by scientists at the USDA and made publicly available in 2006 can be found through the website of the United States Department of Agriculture (USDA) and is discussed in Choi et al. (2007) Genetics 176:685-696. This map was used to locate the flanking SSRs associated with each rust resistance gene and to select SNPs that were mapped within and close to each region. The polymorphism information and genomic sequence on either side of the SNP was used to design PCR-based assays to detect each allele. The sequences with the SNP indicated were either submitted to the Applied Biosystems Inc. (ABI; Foster City, California, United States of America) Assay-by-Design service for creation of custom TAQMAN® (Applied Biosystems Inc., Foster City, California, United States of America) based assays, or assays were manually designed using the ABI software PRIMER EXPRESS®. Similarly, TAQMAN® assays can be designed using software available from Biosearch Technologies (Novato, California, United States of America).

A goal of the SNP assay was to be able to determine which polymorphism(s), or allele(s), is/are present in the genome of any given soybean line, and ultimately to permit the selection of preferred allele(s) (*i*.*e*., rust resistant gene(s)), in a marker-assisted breeding program. A total of 17 SNPs were identified; four for *Rpp1*, five for *Rpp2*, three for *Rpp?*(Hyuuga), two for *Rpp4,* and three for *Rpp5*. A total of 18 screening panel DNAs isolated from resistant lines (PI547875; PI200492; PI594538A; PI368039; PI547878; PI230970; PI224270; PI462312; PI578457A; PI518772; PI628932; PI506764; PI547879; PI459025B; PI200456; PI200526; PI200487; and PI471904) were used for the assays.

### EXAMPLE 3

### Allelic Discrimination Assays

In allelic discrimination assays, a PCR assay included a forward and reverse primer and a specific, fluorescent, dye-labeled probe for each of two alleles for a given

SNP. The probes contained different fluorescent reporter dyes (VIC®; Applied Biosystems, Inc., Foster City, California, United States of America; and 6-carboxyfluorescein-aminohexyl amidite (FAM), or N-TET-6-Aminohexanol (TET) and FAM) to differentiate the amplification of each allele. A non-fluorescent quencher on each probe suppressed the fluorescence until amplification by PCR. During PCR, each probe annealed specifically to complementary sequences between the forward and reverse primer sites. Taq polymerase then cleaved the probes that were hybridized to each allele. Cleavage separated the reporter dye from the quencher, which resulted in increased fluorescence by the reporter dye.

Thus, the fluorescent signals generated by PCR amplification indicated that one or both alleles were present in the sample. In addition to the non-fluorescent quencher, the probes also contained a minor groove binder at the 3' end which resulted in increased melting temperatures (Tm), thereby allowing high specificity with the use of shorter oligos. These probes therefore exhibited greater Tm differences when hybridized to matched and mismatched templates, which provided more accurate allelic discriminations. Probes of this type were manufactured at either ABI (MGB™ quencher) or Biosearch Technologies (BHQPLUS™ quencher). At the end of PCR thermal cycling, fluorescence of the two reporter dyes was measured on an ABI 7900. An increase in fluorescence for one dye only indicated homozygosity for the corresponding allele. An increase in both fluorescent signals indicated heterozygosity.

Exemplary starting lines and haplotypes determined using this method are presented in Tables 3-8. In Tables 3-8, "H" indicates that the line was heterozygous at that position, and "-" indicates that the nucleotide at that position was not determined.

**Table 3**

| SNP Screening Panel | | | | |
|---|---|---|---|---|
| **Line** | **Name** | **Origin** | **Source of Seed** | **Resistance** |
| PI547875 | L85-2378 | Developed in Illinois, USA | GRIN¹ | *Rpp1* |
| PI200492 | | | GRIN | *Rpp1* |
| PI594538A | | | GRIN | *Rppl-b* |
| PI368039 | Tainung No. 4 | | GRIN | *Rpp1* |
| PI547878 | L86-1752 | Developed in Illinois, USA | GRIN | *Rpp2* |
| PI230970 | | | GRIN | *Rpp2* |
| PI224270 | | | GRIN | *Rpp2* |
| PI462312 | Ankur | | GRIN | *Rpp3* |
| PI578457A | | | GRIN | *Rpp3* |
| PI 518772 | | | GRIN | *Rpp3* |
| PI 628932 | | | GRIN | *Rpp3* |
| PI506764 | | | GRIN | *Rpp?* (Hyuuga) |
| PI547879 | L87-0482 | Developed in Illinois, USA | GRIN | *Rpp4* |
| PI459025B | (Bing nan) | | GRIN | *Rpp4* |
| PI200456 | | | GRIN | *Rpp5* |
| PI200526 | | | GRIN | *Rpp5* |
| PI200487 | Kinoshita | | GRIN | *Rpp5* |
| PI471904 | L85-2378 | Developed in Illinois, USA | GRIN | *Rpp5* |

| | | | | |
|---|---|---|---|---|
| ¹Germplasm Resources Information Network, Agricultural Research Service, | | | | |

United States Department of Agriculture, Beltsville, Maryland, United States of America.

**Table 4**

| SNP Genotyping Data - Detailed for *Rpp1* | | | | | | |
|---|---|---|---|---|---|---|
| | | | SEQ ID NO: 1 | SEQ ID NO: 2a | SEQ ID NO: 2b | SEQ ID NO: 3 |
| Linkage Group | | | G | G | G | G |
| Map Position (cM) | | | 100.921 | 101.849 | 101.849 | 103.63 |

| SPIRIT Material ID | ABBRC | LOCUS | *Rpp1* | *Rpp1* | *Rpp1* | *Rpp1* |
|---|---|---|---|---|---|---|
| PI547875 | L85-2378 | *Rpp1* | A | G | T | T |
| PI200492 | | *Rpp1* | A | G | T | T |
| PI594538A | | *Rppl-b* | A | G | T | T |
| PI368039 | Tainung No. 4 | *Rpp1* | A | G | T | T |
| PI547878 | L86-1752 | *Rpp2* | G | A | C | C |
| PI230970 | | *Rpp2* | G | G | C | C |
| PI224270 | | *Rpp2* | G | G | C | C |
| PI462312 | Ankur | *Rpp3* | G | A | C | C |
| PI578457A | | *Rpp3* | A | - | T | C |
| PI 518772 | | *Rpp3* | G | A | C | C |
| PI 628932 | | *Rpp3* | G | A | C | C |
| PI506764 | | *Rpp?* (Hyuuga) | G | G | C | C |
| PI547879 | L87-0482 | *Rpp4* | G | A | C | C |
| PI459025B | (Bing nan) | *Rpp4* | G | G | T | C |
| PI200456 | | *Rpp5* | G | G | T | C |
| PI200526 | | *Rpp5* | H | G | T | C |
| PI200487 | Kinoshita | *Rpp5* | A | G | T | C |
| PI471904 | | *Rpp5* | A | G | T | C |

**Table 5**

| SNP Genotyping Data - Detailed for *Rpp2* | | | | | | | |
|---|---|---|---|---|---|---|---|
| | | | SEQ ID NO: 4a | SEQ ID NO: 4b | SEQ ID NO: 5a | SEQ ID NO: 5b | SEQ ID NO: 6 |
| Lin kage Group | | | J | J | J | J | J |
| Map Position (cM) | | | 49.858 | 49.858 | 57.697 | 57.697 | 60.927 |

| SPIRIT Material ID | ABBRC | LOCUS | *Rpp2* | *Rpp2* | *Rpp2* | *Rpp2* | *Rpp2* |
|---|---|---|---|---|---|---|---|
| PI547875 | L85-2378 | *Rpp1* | G | G | C | T | G |
| PI200492 | | *Rpp1* | G | G | C | T | A |
| PI594538A | | *Rppl-b* | G | G | C | T | A |
| PI368039 | Tainung No. 4 | *Rpp1* | G | G | C | T | A |
| PI547878 | L86-1752 | *Rpp2* | G | G | C | T | G |
| PI230970 | | *Rpp2* | G | | A | T | A |
| PI224270 | | *Rpp2* | G | G | A | A | A |
| PI462312 | Ankur | *Rpp3* | G | G | C | T | H |
| PI578457A | | *Rpp3* | G | G | C | T | A |
| PI 518772 | | *Rpp3* | G | G | H | T | A |
| PI 628932 | | *Rpp3* | G | G | C | T | H |
| PI506764 | | *Rpp?* (Hyuuga) | G | G | A | A | A |
| PI547879 | L87-0482 | *Rpp4* | G | G | C | T | G |
| PI459025B | (Bing nan) | *Rpp4* | G | G | C | T | A |
| PI200456 | | *Rpp5* | G | | A | T | G |
| PI200526 | | *Rpp5* | G | G | H | H | A |
| PI200487 | Kinoshita | *Rpp5* | G | G | A | A | A |
| PI471904 | | *Rpp5* | G | G | A | A | A |

**Table 6**

| SNP Genotyping Data - Detailed for *Rpp3* | | | | | |
|---|---|---|---|---|---|
| | | | SEQ ID NO: 7 | SEQ ID NO: 8a | SEQ ID NO: 8b |
| Linkage Group | | | C2 | C2 | C2 |
| Map Position (cM) | | | 115.71 | 117.9 | 117.9 |

| SPIRIT Material ID | ABBRC | LOCUS | *Rpp3* and *Rpp?*(*Hyuuga*) | *Rpp3* and *Rpp?*(Hyuuga) | *Rpp3* and *Rpp?*(Hyuuga) |
|---|---|---|---|---|---|
| PI547875 | L85-2378 | *Rpp1* | T | T | G |
| PI200492 | | *Rpp1* | C | T | G |
| PI594538A | | *Rppl-b* | C | T | G |
| PI368039 | Tainung No. 4 | *Rpp1* | C | T | G |
| PI547878 | L86-1752 | *Rpp2* | T | T | G |
| PI230970 | | *Rpp2* | C | T | G |
| PI224270 | | *Rpp2* | | C | A |
| PI462312 | Ankur | *Rpp3* | C | T | G |
| PI578457A | | *Rpp3* | C | T | G |
| PI 518772 | | *Rpp3* | T | C | A |
| PI 628932 | | *Rpp3* | C | T | G |
| PI506764 | | *Rpp?* (Hyuuga) | | C | A |
| PI547879 | L87-0482 | *Rpp4* | T | T | G |
| PI459025B | (Bing nan) | *Rpp4* | C | T | G |
| PI200456 | | *Rpp5* | C | T | G |
| PI200526 | | *Rpp5* | C | H | G |
| PI200487 | Kinoshita | *Rpp5* | C | T | G |
| PI471904 | | *Rpp5* | C | T | G |

**Table 7**

| SNP Genotyping Data - Detailed for *Rpp4* | | | | |
|---|---|---|---|---|
| | | | SEQ ID NO: 9 | SEQ ID NO: 10 |
| Linkage Group | | | G | G |
| Map Position (cM) | | | 75.83 | 76.246 |

| SPIRIT Material ID | ABBRC | LOCUS | *Rpp4* | *Rpp4* |
|---|---|---|---|---|
| PI547875 | L85-2378 | *Rpp1* | C | A |
| PI200492 | | *Rpp1* | T | C |
| PI594538A | | *Rppl-b* | T | C |
| PI368039 | Tainung No. 4 | *Rpp1* | T | C |
| PI547878 | L86-1752 | *Rpp2* | C | A |
| PI230970 | | *Rpp2* | T | C |
| PI224270 | | *Rpp2* | C | A |
| PI462312 | Ankur | *Rpp3* | T | A |
| PI578457A | | *Rpp3* | T | C |
| PI 518772 | | *Rpp3* | T | C |
| PI 628932 | | *Rpp3* | C | A |
| PI506764 | | *Rpp?* (Hyuuga) | C | C |
| PI547879 | L87-0482 | *Rpp4* | T | C |
| PI459025B | (Bing nan) | *Rpp4* | T | C |
| PI200456 | | *Rpp5* | T | A |
| PI200526 | | *Rpp5* | C | H |
| PI200487 | Kinoshita | *Rpp5* | T | C |
| PI471904 | | *Rpp5* | T | C |

**Table 8**

| SNP Genotyping Data - Detailed for *Rpp5* | | | | | |
|---|---|---|---|---|---|
| | | | SEQ ID NO: 11 | SEQ ID NO: 12 | SEQ ID NO: 13 |
| Linkage Group | | | N | N | N |
| Map Position (cM) | | | 34.26 | 34.27 | 38.8 |

| SPIRIT Material ID | ABBRC | LOCUS | *Rpp5* | *Rpp5* | *Rpp5* |
|---|---|---|---|---|---|
| PI547875 | L85-2378 | *Rpp1* | A | T | A |
| PI200492 | | *Rpp1* | G | A | G |
| PI594538A | | *Rppl-b* | A | T | G |
| PI368039 | Tainung No. 4 | *Rpp1* | A | T | G |
| PI547878 | L86-1752 | *Rpp2* | A | T | G |
| PI230970 | | *Rpp2* | A | T | A |
| PI224270 | | *Rpp2* | A | T | G |
| PI462312 | Ankur | *Rpp3* | G | A | G |
| PI578457A | | *Rpp3* | | H | G |
| PI 518772 | | *Rpp3* | H | H | H |
| PI 628932 | | *Rpp3* | A | T | A |
| PI506764 | | *Rpp?* (Hyuuga) | A | T | G |
| PI547879 | L87-0482 | *Rpp4* | A | T | G |
| PI459025B | (Bing nan) | *Rpp4* | A | T | A |
| PI200456 | | *Rpp5* | A | T | G |
| PI200526 | | *Rpp5* | G | H | G |
| PI200487 | Kinoshita | *Rpp5* | A | T | G |
| PI471904 | | *Rpp5* | A | T | A |

### EXAMPLE 4

### TAQMAN® Validation

To validate TAQMAN® allelic discrimination assays for association with disease resistance or tolerance, plants were selected based on their known phenotypic status and compared to the genotype at the specific SNP location. DNA isolated from leaf tissue of seedlings 7-10 days after planting was diluted in TE buffer and stored at 4°C until used in PCR reactions as described below.

PCR was set up in 5µl final volumes according to the following formula:

| Reagent | Stock concentration | Per rxn (µl) | For 96 samples (µl) | Final concentration |
|---|---|---|---|---|
| 2X Master Mix* | 2X | 2.5 | 296.88 | 1X |
| AbD primer/probe mix (80x) | 40x | .0625 | 6 | 0.5x |
| PCR-quality H2O | -- | 2.44 | 234.24 | -- |
| DNA (dried in 384) | 4.5 ng/µl | 4 | -- | 3.6 ng/µl (18 ng) |
| Final Volume (µl) | | 5.00 | 357.44 | |

| | | | | |
|---|---|---|---|---|
| *The Master Mix was JUMPSTART™ Taq READYMIX™ (Sigma Catalogue No. 2893; Sigma Chemical Co., St. Louis, Missouri, United States of America), a premix of all the components, including nucleotides and Taq polymerase (but not primers and/or probes) necessary to perform a PCT reaction. Before use, 1375 µl of 1.0 M MgCl₂ (Sigma Catalogue No. M1028) and 250 µl of 300 µM Sulforhodamine 101 (Sigma Catalogue No. S7635; ROX) were added to a 125 mL bottle of JUMPSTART™ Taq READYMIX™. | | | | |

PCR plates were placed in an ABI 9700 thermal cycler and the following program was run: an initial denaturation of 50°C for 2 minutes followed by 95°C for 10 minutes; 40 cycles of 95°C for 15 seconds/60°C for 1 minute; and a final elongation of 72°C for 5 minutes. After the cycling, the samples were incubated at 4°C until needed.

The ABI 7900 Sequence Detection System (or TAQMAN®) was used to visualize the results of an allelic discrimination (SNP) assay. Using the Sequence Detection System (SDS) software, allele calls were made based on the fluorescence for the two dyes measured in each sample.

It will be understood that various details of the presently disclosed subject matter may be changed without departing from the scope of the presently disclosed subject matter. Furthermore, the foregoing description is for the purpose of illustration only, and not for the purpose of limitation.

## Claims

1. Method for detecting the presence of one or more alleles associated with soybean rust (SBR) resistance in a soybean plant or in parts of a soybean plant, comprising detecting the presence of a particular allele of one or more SNPs selected from the group consisting of an allele having an A at nucleotide 428 of SEQ ID NO: 1; a T at position 895 of SEQ ID NO: 2; a G at position 932 of SEQ ID NO: 2; a T at position 57 of SEQ ID NO: 3; a G at position 213 of SEQ ID NO: 4; a G at position 441 of SEQ ID NO: 4; an A at position 70 of SEQ ID NO: 5; a T at position 348 of SEQ ID NO: 5; an A at position 715 of SEQ ID NO: 6; a C at position 377 of SEQ ID NO: 7; a T at position 100 of SEQ ID NO: 8; a G at position 113 of SEQ ID NO: 8; a T at position 147 of SEQ ID NO: 9; a C at position 205 of SEQ ID NO: 10; an A at position 102 at SEQ ID NO: 11; a T at position 159 of SEQ ID NO: 12; and/or a G at position 357 of SEQ ID NO: 13.

2. A method for selecting a soybean rust (SBR) resistant soybean plant, the method comprising:
(a) genotyping one or more soybean plants with respect to one or more single nucleotide polymorphisms (SNPs), wherein the one or more SNPs correspond to least one resistance allele associated with an allele having an A at nucleotide 428 of SEQ ID NO: 1; a T at position 895 of SEQ ID NO: 2; a G at position 932 of SEQ ID NO: 2; a T at position 57 of SEQ ID NO: 3; a G at position 213 of SEQ ID NO: 4; a G at position 441 of SEQ ID NO: 4; an A at position 70 of SEQ ID NO: 5; a T at position 348 of SEQ ID NO: 5; an A at position 715 of SEQ ID NO: 6; a C at position 377 of SEQ ID NO: 7; a T at position 100 of SEQ ID NO: 8; a G at position 113 of SEQ ID NO: 8; a T at position 147 of SEQ ID NO: 9; a C at position 205 of SEQ ID NO: 10; an A at position 102 at SEQ ID NO: 11; a T at position 159 of SEQ ID NO: 12; and/or a G at position 357 of SEQ ID NO: 13; and
(b) selecting a soybean plant that includes at least one resistance allele associated with the one or more SNPs, thereby selecting an SBR resistant soybean plant.

3. A method for selecting a soybean rust (SBR) resistant soybean plant, the method comprising:
(a) isolating one or more nucleic acids from a plurality of soybean plants;
(b) detecting in said isolated nucleic acids the presence of one or more marker molecules associated with SBR resistance, wherein said one or more marker molecules comprises an A at nucleotide 428 of SEQ ID NO: 1; a T at position 895 of SEQ ID NO: 2; a G at position 932 of SEQ ID NO: 2; a T at position 57 of SEQ ID NO: 3; a G at position 213 of SEQ ID NO: 4; a G at position 441 of SEQ ID NO: 4; an A at position 70 of SEQ ID NO: 5; a T at position 348 of SEQ ID NO: 5; an A at position 715 of SEQ ID NO: 6; a C at position 377 of SEQ ID NO: 7; a T at position 100 of SEQ ID NO: 8; a G at position 113 of SEQ ID NO: 8; a T at position 147 of SEQ ID NO: 9; a C at position 205 of SEQ ID NO: 10; an A at position 102 at SEQ ID NO: 11; a T at position 159 of SEQ ID NO: 12; and/or a G at position 357 of SEQ ID NO: 13; and
(c) selecting a soybean plant comprising said one or more marker molecules, thereby selecting an SBR resistant soybean plant.

4. Use of one or more alleles associated with resistance to soybean rust (SBR) selected from an allele having an A at nucleotide 428 of SEQ ID NO: 1; a T at position 895 of SEQ ID NO: 2; a G at position 932 of SEQ ID NO: 2; a T at position 57 of SEQ ID NO: 3; a G at position 213 of SEQ ID NO: 4; a G at position 441 of SEQ ID NO: 4; an A at position 70 of SEQ ID NO: 5; a T at position 348 of SEQ ID NO: 5; an A at position 715 of SEQ ID NO: 6; a C at position 377 of SEQ ID NO: 7; a T at position 100 of SEQ ID NO: 8; a G at position 113 of SEQ ID NO: 8; a T at position 147 of SEQ ID NO: 9; a C at position 205 of SEQ ID NO: 10; an A at position 102 at SEQ ID NO: 11; a T at position 159 of SEQ ID NO: 12; and/or a G at position 357 of SEQ ID NO: 13 for the detection of one or more alleles associated with SBR resistance in a suspected SBR-resistant soybean plant or parts thereof by detecting the presence of the nucleic acid of said one or more allele in said plant or plant part.

5. Use according to claim 4, wherein said SNPs are used in the method of claim 2 or 3 or in a method of producing SBR resistant soybean plants or seeds of SBR resistant soybean plants.

## Patentansprüche

1. Verfahren zum Nachweisen des Vorliegens eines oder mehrerer mit Sojabohnenrost(SBR)-Resistenz assoziierter Allele in einer Sojapflanze oder in Teilen einer Sojapflanze, bei dem man das Vorliegen eines bestimmten Allels eines oder mehrerer SNPs, ausgewählt aus der aus einem Allel mit einem A an Nukleotid 428 von SEQ ID NO: 1; einem T an Position 895 von SEQ ID NO: 2; einem G an Position 932 von SEQ ID NO: 2; einem T an Position 57 von SEQ ID NO: 3; einem G an Position 213 von SEQ ID NO: 4; einem G an Position 441 von SEQ ID NO: 4; einem A an Position 70 von SEQ ID NO: 5; einem T an Position 348 von SEQ ID NO: 5; einem A an Position 715 von SEQ ID NO: 6; einem C an Position 377 von SEQ ID NO: 7; einem T an Position 100 von SEQ ID NO: 8; einem G an Position 113 von SEQ ID NO: 8; einem T an Position 147 von SEQ ID NO: 9; einem C an Position 205 von SEQ ID NO: 10; einem A an Position 102 von SEQ ID NO: 11; einem T an Position 159 von SEQ ID NO: 12; und/oder einem G an Position 357 von SEQ ID NO: 13 bestehenden Gruppe, nachweist.

2. Selektionsverfahren für eine gegen Sojabohnenrost (SBR) resistente Sojapflanze, bei dem man:
(a) eine oder mehrere Sojapflanzen in Bezug auf einen oder mehrere Einzelnukleotid-Polymorphismen (SNPs) genotypisiert, wobei der eine SNP bzw. die mehreren SNPs wenigstens einem mit einem Allel mit einem A an Nukleotid 428 von SEQ ID NO: 1; einem T an Position 895 von SEQ ID NO: 2; einem G an Position 932 von SEQ ID NO: 2; einem T an Position 57 von SEQ ID NO: 3; einem G an Position 213 von SEQ ID NO: 4; einem G an Position 441 von SEQ ID NO: 4; einem A an Position 70 von SEQ ID NO: 5; einem T an Position 348 von SEQ ID NO: 5; einem A an Position 715 von SEQ ID NO: 6; einem C an Position 377 von SEQ ID NO: 7; einem T an Position 100 von SEQ ID NO: 8; einem G an Position 113 von SEQ ID NO: 8; einem T an Position 147 von SEQ ID NO: 9; einem C an Position 205 von SEQ ID NO: 10; einem A an Position 102 von SEQ ID NO: 11; einem T an Position 159 von SEQ ID NO: 12; und/oder einem G an Position 357 von SEQ ID NO: 13 assoziierten Resistenzallel entspricht bzw. entsprechen; und
(b) eine Sojapflanze, die wenigstens ein mit dem einen SNP bzw. den mehreren SNPs assoziiertes Resistenzallel enthält, und damit eine SBR-resistente Sojapflanze selektioniert.

3. Selektionsverfahren für eine gegen Sojabohnenrost (SBR) resistente Sojapflanze, bei dem man:
(a) eine oder mehrere Nukleinsäuren aus mehreren Sojapflanzen isoliert;
(b) in den isolierten Nukleinsäuren das Vorliegen eines oder mehrerer mit SBR-Resistenz assoziierter Marker-Moleküle nachweist, wobei das eine Marker-Molekül bzw. die mehreren Marker-Moleküle ein A an Nukleotid 428 von SEQ ID NO: 1; ein T an Position 895 von SEQ ID NO: 2; ein G an Position 932 von SEQ ID NO: 2; ein T an Position 57 von SEQ ID NO: 3; ein G an Position 213 von SEQ ID NO: 4; ein G an Position 441 von SEQ ID NO: 4; ein A an Position 70 von SEQ ID NO: 5; ein T an Position 348 von SEQ ID NO: 5; ein A an Position 715 von SEQ ID NO: 6; ein C an Position 377 von SEQ ID NO: 7; ein T an Position 100 von SEQ ID NO: 8; ein G an Position 113 von SEQ ID NO: 8; ein T an Position 147 von SEQ ID NO: 9; ein C an Position 205 von SEQ ID NO: 10; ein A an Position 102 von SEQ ID NO: 11; ein T an Position 159 von SEQ ID NO: 12; und/oder ein G an Position 357 von SEQ ID NO: 13 umfasst bzw. umfassen; und
(c) eine das eine Marker-Molekül bzw. die mehreren Marker-Moleküle enthaltende Sojapflanze und damit eine SBR-resistente Sojapflanze selektioniert.

4. Verwendung eines oder mehrerer mit Resistenz gegen Sojabohnenrost (SBR) assoziierter Allele, ausgewählt aus einem Allel mit einem A an Nukleotid 428 von SEQ ID NO: 1; einem T an Position 895 von SEQ ID NO: 2; einem G an Position 932 von SEQ ID NO: 2; einem T an Position 57 von SEQ ID NO: 3; einem G an Position 213 von SEQ ID NO: 4; einem G an Position 441 von SEQ ID NO: 4; einem A an Position 70 von SEQ ID NO: 5; einem T an Position 348 von SEQ ID NO: 5; einem A an Position 715 von SEQ ID NO: 6; einem C an Position 377 von SEQ ID NO: 7; einem T an Position 100 von SEQ ID NO: 8; einem G an Position 113 von SEQ ID NO: 8; einem T an Position 147 von SEQ ID NO: 9; einem C an Position 205 von SEQ ID NO: 10; einem A an Position 102 von SEQ ID NO: 11; einem T an Position 159 von SEQ ID NO: 12; und/oder einem G an Position 357 von SEQ ID NO: 13, zum Nachweis eines oder mehrerer mit SBR-Resistenz assoziierter Allele in einer mutmaßlich SBR-resistenten Sojapflanze oder Teilen davon durch Nachweisen des Vorliegens der Nukleinsäure des einen Allels bzw. der mehreren Allele in der Pflanze bzw. dem Pflanzenteil.

5. Verwendung nach Anspruch 4, wobei die SNPs bei dem Verfahren gemäß Anspruch 2 oder 3 oder bei einem Verfahren zur Herstellung SBR-resistenter Sojapflanzen oder von Samen SBR-resistenter Sojapflanzen verwendet werden.

## Revendications

1. Procédé de détection de la présence d'un ou plusieurs allèles associés à la résistance à la rouille du soja (SBR) dans une plante de soja ou dans des parties d'une plante de soja, comprenant la détection de la présence d'un allèle particulier d'un ou plusieurs SNP choisis dans le groupe constitué d'un allèle ayant un A au nucléotide 428 de SEQ ID NO: 1 ; un T à la position 895 de SEQ ID NO: 2 ; un G à la position 932 de SEQ ID NO: 2 ; un T à la position 57 de SEQ ID NO: 3 ; un G à la position 213 de SEQ ID NO: 4 ; un G à la position 441 de SEQ ID NO: 4 ; un A à la position 70 de SEQ ID NO: 5 ; un T à la position 348 de SEQ ID NO: 5 ; un A à la position 715 de SEQ ID NO: 6 ; un C à la position 377 de SEQ ID NO: 7 ; un T à la position 100 de SEQ ID NO: 8 ; un G à la position 113 de SEQ ID NO: 8 ; un T à la position 147 de SEQ ID NO: 9 ; un C à la position 205 de SEQ ID NO: 10 ; un A à la position 102 de SEQ ID NO: 11 ; un T à la position 159 de SEQ ID NO: 12 ; et/ou un G à la position 357 de SEQ ID NO: 13.

2. Procédé de sélection d'une plante de soja résistante à la rouille du soja (SBR), le procédé comprenant :
(a) le génotypage d'une ou plusieurs plantes de soja concernant un ou plusieurs polymorphismes de nucléotide unique (SNP), les un ou plusieurs SNP correspondant à au moins un allèle de résistance associé à un allèle ayant un A au nucléotide 428 de SEQ ID NO: 1 ; un T à la position 895 de SEQ ID NO: 2 ; un G à la position 932 de SEQ ID NO: 2 ; un T à la position 57 de SEQ ID NO: 3 ; un G à la position 213 de SEQ ID NO: 4 ; un G à la position 441 de SEQ ID NO: 4 ; un A à la position 70 de SEQ ID NO: 5 ; un T à la position 348 de SEQ ID NO: 5 ; un A à la position 715 de SEQ ID NO: 6 ; un C à la position 377 de SEQ ID NO: 7 ; un T à la position 100 de SEQ ID NO: 8 ; un G à la position 113 de SEQ ID NO: 8 ; un T à la position 147 de SEQ ID NO: 9 ; un C à la position 205 de SEQ ID NO: 10 ; un A à la position 102 de SEQ ID NO: 11 ; un T à la position 159 de SEQ ID NO: 12 ; et/ou un G à la position 357 de SEQ ID NO: 13 ;
et
(b) la sélection d'une plante de soja qui comprend au moins un allèle de résistance associé aux un ou plusieurs SNP, de manière à sélectionner une plante de soja résistante à SBR.

3. Procédé de sélection d'une plante de soja résistante à la rouille du soja (SBR), le procédé comprenant :
(a) l'isolement d'un ou plusieurs acides nucléiques d'une pluralité de plantes de soja ;
(b) la détection dans lesdits acides nucléiques isolés de la présence d'une ou plusieurs molécules marqueuses associées à la résistance à SBR, lesdites une ou plusieurs molécules marqueuses comprenant un A au nucléotide 428 de SEQ ID NO: 1 ; un T à la position 895 de SEQ ID NO: 2 ; un G à la position 932 de SEQ ID NO: 2 ; un T à la position 57 de SEQ ID NO: 3 ; un G à la position 213 de SEQ ID NO: 4 ; un G à la position 441 de SEQ ID NO: 4 ; un A à la position 70 de SEQ ID NO: 5 ; un T à la position 348 de SEQ ID NO: 5 ; un A à la position 715 de SEQ ID NO: 6 ; un C à la position 377 de SEQ ID NO: 7 ; un T à la position 100 de SEQ ID NO: 8 ; un G à la position 113 de SEQ ID NO: 8 ; un T à la position 147 de SEQ ID NO: 9 ; un C à la position 205 de SEQ ID NO: 10 ; un A à la position 102 de SEQ ID NO: 11 ; un T à la position 159 de SEQ ID NO: 12 ; et/ou un G à la position 357 de SEQ ID NO: 13 ; et
(c) la sélection d'une plante de soja comprenant lesdites une ou plusieurs molécules marqueuses, de manière à sélectionner une plante de soja résistante à SBR.

4. Utilisation d'un ou plusieurs allèles associés à la résistance à la rouille du soja (SBR) choisis parmi un allèle ayant un A au nucléotide 428 de SEQ ID NO: 1 ; un T à la position 895 de SEQ ID NO: 2 ; un G à la position 932 de SEQ ID NO: 2 ; un T à la position 57 de SEQ ID NO: 3 ; un G à la position 213 de SEQ ID NO: 4 ; un G à la position 441 de SEQ ID NO: 4 ; un A à la position 70 de SEQ ID NO: 5 ; un T à la position 348 de SEQ ID NO: 5 ; un A à la position 715 de SEQ ID NO: 6 ; un C à la position 377 de SEQ ID NO: 7 ; un T à la position 100 de SEQ ID NO: 8 ; un G à la position 113 de SEQ ID NO: 8 ; un T à la position 147 de SEQ ID NO: 9 ; un C à la position 205 de SEQ ID NO: 10 ; un A à la position 102 de SEQ ID NO: 11 ; un T à la position 159 de SEQ ID NO: 12 ; et/ou un G à la position 357 de SEQ ID NO: 13 pour la détection d'un ou plusieurs allèles associés à la résistance à SBR dans une plante de soja suspectée d'être résistante à SBR ou des parties de celle-ci par détection de la présence de l'acide nucléique desdits un ou plusieurs allèles dans ladite plante ou partie de plante.

5. Utilisation selon la revendication 4, dans laquelle lesdits SNP sont utilisés dans le procédé de la revendication 2 ou 3 ou dans un procédé de production de plantes de soja résistantes à SBR ou des semences de plantes de soja résistantes à SBR.
